# EUROPEAN PATENT SPECIFICATION

(11) **EP 3 528 829 B1**
(45) Date of publication and mention of the grant of the patent: **02.03.2022**
(21) Application number: 17787188.6
(22) Date of filing: 20.10.2017
(51) Int. Cl.: A61K 38/12, A61K 31/715, A61K 47/50, A61P 31/04

(54) **BACITRACIN-ALGINATE OLIGOMER CONJUGATES**
BACITRACIN-ALGINAT OLIGOMER KONJUGATE
CONJUGÉS BACITRACINE-ALGINATE OLIGOMÉRIQUE

(30) Priority: 21.10.2016 GB 201617862
(43) Date of publication of application: 28.08.2019
(73) Proprietor: Algipharma AS, 1337 Sandvika (NO)
(72) Inventor: FERGUSON, Elaine, Cardiff South Wales CF14 4XY (GB); THOMAS, David William, Cardiff South Wales CF14 4XY (GB); DESSEN, Arne, 3440 Røyken (NO); RYE, Philip, 1359 Eiksmarka (NO)
(74) Representative: Dehns
(86) International application number: PCT/EP2017/076926
(87) International publication number: WO 2018/073448

(56) References cited:
- WO-A1-2010/139956
- US-A- 3 952 092
- US-A1- 2014 364 595

## Description

The present invention provides a novel modified form of bacitracin-class antibiotics having advantageous properties. In particular, it has been found that by providing bacitracin-class antibiotics as conjugates with alginate oligomers, the spectrum of activity of the antibiotic, which typically in unconjugated form is restricted to gram-positive bacteria, may surprisingly be broadened to include gram-negative bacteria. The invention is accordingly directed to providing medical uses and methods using bacitracin-alginate oligomer conjugates in the treatment or prevention of bacterial infections. The invention further provides methods for preparing the conjugates of the invention.

Antibiotics effective against Gram negative bacteria are known and it has been shown that their efficacy can be enhanced by the addition of alginate oligomers fragments (see WO 2010/139956 A1).

However, the therapeutic use of known bacitracin-class antibiotics is presently restricted to the treatment of infections with Gram positive bacteria because such antibiotics have only very limited, if any, efficacy against Gram negative bacteria. Any effectiveness against Gram negative bacteria which may be observed experimentally is typically so slight that the amounts required to exert a therapeutic effect would give rise to unacceptable side effects. It has now been observed that covalently conjugating alginate oligomers to bacitracin-class antibiotics widens the spectrum of therapeutically useful activity against bacteria species such to include Gram negative bacteria. In other words bacitracin-alginate oligomer conjugates are a class of novel chemical entities having therapeutically useful antibacterial efficacy against both Gram negative and Gram positive bacteria. The bacitracin-alginate oligomer conjugates of the invention therefore represent improved therapeutic agents for the treatment or prevention of bacterial infections in animal subjects. Bacitracin-class antibiotics are a well-known and well-characterised class of cyclic polypeptide antibiotics that inhibit synthesis of the bacterial cell wall (Ming, L-J., et al., 2002, Journal of Inorganic Chemistry, Vol 91(1):46-58, which is herein incorporated by reference in its entirety). Bacitracin was initially isolated from cultures of *Bacillus subtilis* and *Bacillus licheniformis* and it has since been found that this isolate is a complex mixture of structurally similar dodecapeptide congeners. The most prevalent congeners in such isolates have to date been termed bacitracin A1, A2, B1, B2, B3, C, D1, D2 ,D3, E, F, G, H1, H2, H3, I1, I2, I3, and X. Bacitracin A1, A2, B1, B2, B3, C, D1, D2, D3 and E are considered to have the greatest antibiotic efficacy and natural bacitracin preparations are typically comprised predominantly of bacitracin A and bacitracin B.

The bacitracin-class antibiotics maybe described generally as branched cyclic dodecapeptides, more specifically heptacyclic peptides having a pentapeptide side chain, which demonstrate antibiotic efficacy, believed to be primarily by virtue of the inhibition of bacterial cell wall synthesis. More specifically, it is believed that bacitracin-class antibiotics bind undecaprenyl pyrophosphate which prevents the recycling of this sugar carrier during peptidoglycan synthesis and this in turn inhibits bacterial cell wall synthesis. Gram positive species may have cell walls containing as much as 90% peptidoglycan whereas Gram negative species may have as little as 10%. This is believed to explain bacitracin's apparent selective antibacterial effects against Gram negative and Gram positive species. Antibacterial activity is dependent on complexing with divalent metal cations, e.g. Zn²⁺, Mg²⁺, Mn²⁺, and Co²⁺. As such, derivatives carrying a variety of metal chelating groups have been proposed (WO 97/47313).

Functionally, the class is very effective against Gram positive bacteria, especially Gram-positive bacilli and cocci including *Staphylococcus, Streptococcus, Bacillus, Micrococcus* and *Clostridium.* In contrast, efficacy against Gram negative bacteria is very limited and usually to the extent that the amounts required for therapeutic effectiveness are associated with typically unacceptable side effects. Systemic use is however associated with toxicity issues (that is toxicity to the subject or "host" to whom the bacitracin-class antibiotic is administered, namely "host toxicity"), in particular nephrotoxicity. Localised topical use and oral use to treat GI infections (bacitracin is poorly absorbed from the GI tract) is less therefore less problematic.

There is an on-going need for new antibiotics and also ways to make existing antibiotics more flexible to use.

Alginate oligomers have been described in the literature at length. Briefly, alginates are linear polymers of (1-4) linked β-D-mannuronic acid (M) and/or its C-5 epimer α-L-guluronic acid (G). The primary structure of alginates can vary greatly. The M and G residues can be organised as homopolymeric blocks of contiguous M or G residues, as blocks of alternating M and G residues and single M or G residues can be found interspacing these block structures. An alginate molecule can comprise some or all of these structures and such structures might not be uniformly distributed throughout the polymer. In the extreme, there exists a homopolymer of guluronic acid (polyguluronate) or a homopolymer of mannuronic acid (polymannuronate). Alginate oligomers may be obtained from alginate polymers which are typically isolated from natural sources as large high molecular weight polymers (e.g. an average molecular weight in the range 300,000 to 500,000 Daltons). Such large alginate polymers may be degraded, or broken down, e.g. by chemical or enzymatic hydrolysis to produce alginate structures of lower molecular weight.

As shown in the Examples, it has now been found that covalent conjugation of bacitracin-class antibiotics to alginate oligomers creates a novel chemical entity with antibacterial efficacy against Gram-negative bacteria.

Accordingly, in a first aspect the invention provides a bacitracin-alginate oligomer conjugate comprising a bacitracin-class antibiotic connected covalently to at least one alginate oligomer via a direct covalent bond or a covalent molecular linker, wherein the alginate oligomer has 2 to 50 monomer residues.

The bacitracin-alginate oligomer conjugates may also be described by Formula I:

B-(L-A)ₙ ( I)

wherein B- is a bacitracin-class antibiotic, L is a direct covalent bond or a covalent molecular linker, -A is an alginate oligomer and n is an integer of 1 to 10, e.g. 1 to 9, 1 to 8, 1 to 7, 1 to 6, 1 to 5, 1 to 4, 1 to 3, or 2 or 1.

In accordance with the invention a bacitracin-class antibiotic is broadly defined as a cyclic dodecapeptide which demonstrates antibacterial efficacy wherein said peptide consists of a heptacyclic peptide with a pentapeptide side chain. Specifically contemplated herein are naturally occurring bacitracins or functionally equivalent derivatives thereof which retain antibacterial efficacy, including fully and semi-synthetic forms. Thus included with the term bacitracin-class antibiotic are bacitracin A1, A2, B1, B2, B3, C, D1, D2 ,D3, E, F, G, H1, H2, H3, I1, 12, 13, and X, e.g. as described, *inter alia*, in Ming, L-J., et al., *supra*, and Economou, NJ,. et al, 2013, Vol 110, 14207-14212. Functionally equivalent derivatives are described in, *inter alia*, WO 97/47313, WO 2011/051073 and WO 2011/051071.

By way of example a bacitracin-class antibiotic may be represented by Formula II 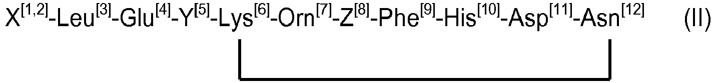
wherein Leu is leucine; Glu is glutamic acid; Lys is lysine; Orn is ornithine; Phe is phenylalanine; His is histidine; Asp is aspartic acid; Asn is asparagine;
Y is valine, isoleucine, leucine or 5-methylene-isoleucine;
Z is valine, isoleucine, leucine or 5-methylene-isoleucine; and
X is W^{[1]}-Cys^{[2]} or V^{[1]}-Thz^{[2]}; wherein
   W is valine, isoleucine, leucine or 5-methylene-isoleucine and Cys is cysteine; and
   V is

      H₂N-C(R)H-

      wherein R is the α side chain of valine, isoleucine, leucine or 5-methylene-isoleucine; and

Thz is a thiazoline ring 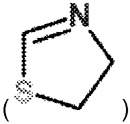 which is 2' coupled to V and 4' coupled to the α-carbon of Leu^{[3]}.

In Formula II the ε-amine of Lys^{[6]} is coupled to the α-carboxyl group of Asn^{[12]} by a peptide bond.

In accordance with the invention references to bacitracin-class antibiotics extend to antibacterial derivatives of Formula II, i.e. functionally equivalent derivatives which retain (e.g. have at least 70%, 80%, 90% or 95% of the) antibacterial efficacy of bacitracin A1 and/or A2, wherein one or more, of amino acids Leu^{[3]}, Glu^{[4]}, Orn^{[7]}, Phe^{[9]}, His^{[10]} or Asp^{[11}is replaced by another amino acid residue which may be selected from natural or non-genetically encoded amino acids, e.g. leucine, threonine, acid, phenylalanine, arginine, histidine, lysine, asparagine, serine, cysteine, homolysine, ornithine, diaminobutyric acid (e.g. α,γ-diaminobutyric acid), diaminopimelic acid, diaminopropionic acid, homoarginine, trimethylysine, trimethylornithine, 4-aminopiperidine-4-carboxylic acid, 4-amino-1-carbamimidoylpiperidine-4-carboxylic acid and 4-guanidinophenylalanine.

In accordance with the invention references to bacitracin-class antibiotics extend to antibacterial equivalents of Formula II, i.e. functional equivalents which retain (e.g. have at least 70%, 80%, 90% or 95% of the) antibacterial efficacy of bacitracin A1 and/or A2, wherein Thz is substituted with a fluorine, chlorine or bromine, or a linear or branched aliphatic unsaturated or saturated C₁-C₄ alkyl or alkoxy group, e.g. methyl, ethyl, propyl, iso-propyl, butyl, iso-butyl, sec-butyl, tert-butyl, ethylene, propylene, butylene, hydroxy, methoxy, ethyloxy, propyloxy, iso-propyloxy, butyloxy group, iso-butyloxy, sec-butyloxy, tert-butyloxy or halogen substituted versions thereof.

Preferably the substituting amino acid is an amino acid with a cationic side chain, i.e. an amino acid that has a side chain that has a net positive charge at the intracellular pH of a tumour cell, e.g. around pH 7.4. Of the genetically coded amino acids this would include lysine and arginine but any non-genetically coded or modified amino acid carrying such a net positive charge on its side chain may be used, e.g. those amino acids carrying a side-chain with a guanidino group or an amine group or another cationic moiety, e.g. derivatives of lysine, and arginine in which any hydrogen in the side chain, except the protonating hydrogen, is substituted with a halogen atom, e.g. fluorine, chlorine or bromine, or a linear, branched aliphatic unsaturated or saturated C₁-C₄ alkyl or alkoxy group, e.g. methyl, ethyl, propyl, iso-propyl, butyl, iso-butyl, sec-butyl, tert-butyl, ethylene, propylene, butylene, hydroxy, methoxy, ethyloxy, propyloxy, iso-propyloxy, butyloxy group, iso-butyloxy, sec-butyloxy, tert-butyloxy or halogen substituted versions thereof. Suitable non-genetically coded amino acids with cationic side chains include homolysine, ornithine, diaminobutyric acid, diaminopimelic acid, diaminopropionic acid and homoarginine as well as trimethylysine and trimethylornithine, 4-aminopiperidine-4-carboxylic acid, 4-amino-1-carbamimidoylpiperidine-4-carboxylic acid and 4-guanidinophenylalanine.

An amino acid is a molecule containing an amine group, a carboxylic acid group and at least one carbon separating these two groups. Other groups may be attached to the separating carbon(s). These groups may be referred to as "side-chains" although at its most simple the side chains could be hydrogen (glycine). Amino acids with a single separating carbon are termed "a-amino acids" and have the generic formula H₂NCR₁R₂COOH, where R₁ and R₂ are substituent groups, i.e. are side-chains. The separating carbon is known as the α-carbon. Other types of amino acid exist where the amino and carboxylic acid groups are separated by more than a single carbon atom; for example, in β-amino acids the carbon atom to which the amino group is separated from the carboxylic acid group by two carbon atoms and in γ-amino acids three carbon atoms separate the amino and carboxylic acid groups. Preferably the amino acids in the bacitracin-class antibiotic of use in the invention will be α, β or γ-amino acids, more preferably α or β-amino acids and most preferably α-amino acids.

Amino acids, with the exception of glycine, may exist as two or more stereoisomers. In particular the α-carbon of an amino acid other than glycine is a chiral centre and so gives rise to two enantiomeric forms of each amino acid. These forms are often referred to as D and L forms, e.g. D-alanine and L-alanine. Amino acids with further chiral centres will exist in four or more possible stereoisomers, e.g. threonine has two chiral centres and so may exist in one of four stereoisomeric forms. Any stereoisomeric form of an amino acid may be present the bacitracin-class antibiotic molecules of use in the invention. For the purposes of describing the present invention, where the term "non-genetically encoded" is applied to amino acids, this does not include the D forms of amino acids that occur in nature in the L form.

In preferred embodiments the bacitracin-class antibiotic is selected from bacitracin A1, A2, B1, B2, B3, C, D1, D2, D3, E, F, G, H1, H2, H3, I1, I2, I3, and X, more preferably selected from A1, A2, B1, B2, B3, C, D1, D2, D3 and E, and more preferably from bacitracin A (A1 and/or A2) and bacitracin B (B1 and/or B2). These bacitracins may be represented by Formula III: 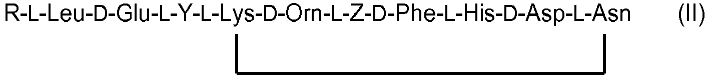 wherein

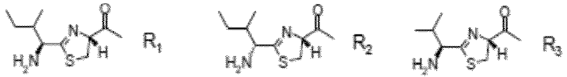

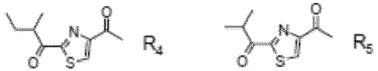

| Bacitracin | R | L-Y | L-Z |
|---|---|---|---|
| A1 | R₁ | Ile | Ile |
| A2 | R₂ | Ile | Ile |
| B1 | R₃ | Ile | Ile |
| B2 | R₁ | Val | Ile |
| B3 | R₁ | Ile | Val |
| D1 | R₃ | Val | Ile |
| D2 | R₃ | Ile | Val |
| D3 | R₁ | Val | Val |
| E | R₃ | Val | Val |
| F | R₄ | Ile | Ile |
| H1 | R₅ | Ile | Ile |
| H2 | R₄ | Val | Ile |
| H3 | R₄ | Ile | Val |
| I1 | R₅ | Val | Ile |
| I2 | R₅ | Ile | Val |
| I3 | R₄ | Val | Val |

In accordance with the invention preferred bacitracin-class antibiotics are functionally equivalent derivatives of the bacitracins mentioned immediately above i.e. functional equivalents which retain (e.g. have at least 70%, 80%, 90% or 95% of the) the antibacterial efficacy of the bacitracin in question.

In certain embodiments the bacitracin-class antibiotic may be complexed with divalent metal cations, e.g. Zn²⁺, Mg²⁺, Mn²⁺, and Co²⁺.

In certain embodiments one or more free amine groups in the bacitracin-class antibiotic may be masked by modification, e.g. by sulfomethylation.

As noted above, alginates typically occur as polymers of an average molecular mass of at least 35,000 Daltons, i.e. approximately 175 to approximately 190 monomer residues, although typically much higher. An alginate oligomer according to the present invention will, on the other hand, contain 2 to 50 monomer residues, more typically 3, 4, 5 or 6 to 50, and may contain 2, 3, 4, 5 or 6 to 50, 2, 3, 4, 5 or 6 to 40, 2, 3, 4, 5 or 6 to 35 or 2, 3, 4, 5 or 6 to 30 residues. Thus, an alginate oligomer for use according to the invention will typically have an average molecular weight of 350, 550, 700, 900 or 1000 to 10,000 Daltons, 350, 550, 700, 900 or 1000 to 8000 Daltons, 350, 550, 700, 900 or 1000 to 7000 Daltons, or 350, 550, 700, 900 or 1000 to 6,000 Daltons.

Alternatively put, the alginate oligomer may have a degree of polymerisation (DP), or a number average degree of polymerisation (DPn) of 2 to 50, preferably 2 to 40, 2 to 35, 2 to 30, 2 to 28, 2 to 25, 2 to 22, 2 to 20, 2 to 18, 2 to 17, 2 to 15 or 2 to 12.

Other representative ranges (whether for the number of residues, DP or DPn) include any one of 3, 4, 5, 6, 7, 8, 9, 10 or 11 to any one of 50, 45, 40, 39, 38, 37, 36, 35, 34, 33, 32, 31, 30, 29, 28, 27, 26, 25, 24, 23, 22, 21, 20, 19, 18, 17, 16, 15, 14, 13 or 12.

Other representative ranges (whether for the number of residues, DP or DPn) include any one of 8, 9, 10, 11, 12, 13, 14 or 15 to any one of 50, 45, 40, 39, 38, 37, 36, 35, 34, 33, 32, 31, 30, 29, 28, 27, 26, 25, 24, 23, 22, 21, 20, 19, 18, 17 or 16.

Other representative ranges (whether for the number of residues, DP or DPn) include any one of 11, 12, 13, 14, 15, 16, 17 or 18 to any one of 50, 45, 40, 39, 38, 37, 36, 35, 34, 33, 32, 31, 30, 29, 28, 27, 26, 25, 24, 23, 22, 21, 20 or 19.

It may in some embodiments be advantageous to select a larger alginate oligomer so as to create a conjugate of greater size. Larger conjugates may help deliver the bacitracin-class antibiotics selectively to sites and locations of infection because the vascular permeability of such areas within a subject is typically greater than in the vasculature of non-infected areas. Consequently larger conjugates are less likely to enter non-infected areas from the blood stream, but would able to enter the more permeable infected areas. A representative size range for such a larger oligomer may for example be 20 to 50 residues (or DP or DPn of 20 to 50) or any one of 20, 21, 22, 23, 24 or 25, to any one of 50, 45, 40, 35 or 30 residues (or DP or DPn of any one of these ranges) or any one of 30, 31, 32, 33, 34 or 35, to any one of 50, 45 or 40 residues (or DP or DPn of any one of these ranges). Alternatively, these results might be also be achieved by increasing the numbers of alginate oligomers, even those of smaller size, in the conjugate.

An alginate oligomer will, as noted above, contain (or comprise) guluronate or guluronic acid (G) and/or mannuronate or mannuronic acid (M) residues or units. An alginate oligomer according to the invention will preferably be composed solely, or substantially solely (i.e. consist essentially of) uronate/uronic acid residues, more particularly solely or substantially solely of G and/or M residues. Alternatively expressed, in the alginate oligomer of use in the present invention, at least 80%, more particularly at least 85, 90, 95 or 99% of the monomer residues may be uronate/uronic acid residues, or, more particularly G and/or M residues. In other words, preferably the alginate oligomer will not comprise other residues or units (e.g. other saccharide residues, or more particularly other uronic acid/uronate residues).

The alginate oligomer is preferably a linear oligomer.

More particularly, the alginate oligomers proposed for use according to the present invention will contain at least 70% G residues (i.e. at least 70% of the monomer residues of the alginate oligomer will be G residues). Specific embodiments thus include alginate oligomers with (e.g. containing) 70 to 100% G (guluronate) residues.

Preferably at least 75% or 80%, more particularly at least 85% or 90%, even more particularly at least 91, 92, 93, 94, 95, 96, 97, 98 or 99% of the monomer residues are guluronate. In one embodiment the alginate oligomer may be an oligoguluronate (i.e. a homooligomer of G, or 100% G).

In a further preferred embodiment, the above described alginates of the invention have a primary structure wherein the majority of the G residues are in so called G-blocks. Preferably at least 50%, more preferably at least 70 or 75%, and most preferably at least 80, 85, 90, 92 or 95% of the G residues are in G-blocks. A G block is a contiguous sequence of at least two G residues, preferably at least 3 contiguous G residues, more preferably at least 4 or 5 contiguous G residues, most preferably at least 7 contiguous G residues.

In particular at least 90% of the G residues are linked 1-4 to another G residue. More particularly at least 95%, more preferably at least 98%, and most preferably at least 99% of the G residues of the alginate are linked 1-4 to another G residue. More specifically at least 70% of the monomer residues in the oligomer are G residues linked 1-4 to another G-residue, or more preferably at least 75%, and most preferably at least 80, 85, 90, 92, 93, 94, 95, 96, 97, 98, 99% of the monomers residues of the oligomer are G residues linked 1-4 to another G residue. This 1-4 linkage of two G residues can be alternatively expressed as a guluronic unit bound to an adjacent guluronic unit.

The alginate oligomers of use in the invention are commonly referred to by the skilled person as "high G" or "G-block" oligomers i.e. having a high content of G residues or G-blocks (e.g. wherein at least 70% of the monomer residues are G, preferably arranged in G-blocks).

The alginate oligomer of use in the invention is preferably a 3- to 35-mer, more preferably a 3- to 28-mer, in particular a 4- to 25-mer, e.g. a 5- to 20-mer, especially a 6- to 22-mer, in particular an 8- to 20-mer, especially a 10- to 15-mer, e.g. having a molecular weight in the range 350 to 6400 Daltons or 350 to 6000 Daltons, preferably 550 to 5500 Daltons, preferably 750 to 5000 Daltons, and especially 750 to 4500 Daltons or 2000 to 3000 Daltons or 900 to 3500 Daltons. Other representative alginate oligomers include, as mentioned above, oligomers with 5, 6, 7, 8, 9, 10, 11, 12 or 13 to 50, 45, 40, 35, 28, 25, 22 or 20 residues.

It may be a single compound or it may be a mixture of compounds, e.g. of a range of degrees of polymerization. As noted above, the monomeric residues in the alginate oligomer, may be the same or different and not all need carry electrically charged groups although it is preferred that the majority (e.g. at least 60%, preferably at least 80% more preferably at least 90%) do. It is preferred that a substantial majority, e.g. at least 80%, more preferably at least 90% of the charged groups have the same polarity. In the alginate oligomer, the ratio of hydroxyl groups to charged groups is preferably at least 2:1, more especially at least 3:1.

The alginate oligomer of the invention may have a degree of polymerisation (DP), or a number average degree of polymerisation (DPₙ), of 3-28, 4-25, 6-22, 8-20 or 10-15, or 5-18 or 7-15 or 8-12, especially 10.

The alginate oligomer of the invention may have a degree of polymerisation (DP), or a number average degree of polymerisation (DPₙ), of 3-24, 4-23, 5-22, 6-21, 7-20, 8-19, 9-18, 10-17, 11-16, 12-15 or 13-14 (e.g. 13 or 14).

The alginate oligomer of the invention may have a degree of polymerisation (DP), or a number average degree of polymerisation (DPn), of 4-25, 5-24, 6-23, 7-22, 8-21, 9-20, 10-19, 11-18, 12-17, 13-16, 14-15 (e.g. 14 or 15).

The alginate oligomer of the invention may have a degree of polymerisation (DP), or a number average degree of polymerisation (DPₙ), of 5-26, 6-25, 7-24, 8-23, 9-22, 10-21, 11-20, 12-19, 13-18, 14-17 or 15-16 (e.g. 15 or 16).

The alginate oligomer of the invention may have a degree of polymerisation (DP), or a number average degree of polymerisation (DPₙ), of 4-50, 4-40, 4-35, 4-30, 4-28, 4-26, 4-22, 4-20, 4-18, 4-16 or 4-14.

The alginate oligomer of the invention may have a degree of polymerisation (DP), or a number average degree of polymerisation (DPₙ), of 5-50, 5-40, 5-25, 5-22, 5-20, 5-18, 5-23, 5-20, 5-18, 5-16 or 5-14.

The alginate oligomer of the invention may have a degree of polymerisation (DP), or a number average degree of polymerisation (DPₙ), of 6-50, 6-40, 6-35, 6-30, 6-28, 6-26, 6-24, 6-20, 6-19, 6-18, 6-16 or 6-14.

The alginate oligomer of the invention may have a degree of polymerisation (DP), or a number average degree of polymerisation (DPₙ), of 8-50, 8-40, 8-35, 8-30, 8-28, 8-25, 8-22, 8-20, 8-18, 8-16 or 8-14.

The alginate oligomer of the invention may have a degree of polymerisation (DP), or a number average degree of polymerisation (DPₙ), of 9-50, 9-40, 9-35, 9-30, 9-28, 9-25, 9-22, 9-20, 9-18, 9-16 or 9-14.

The alginate oligomer of the invention may have a degree of polymerisation (DP), or a number average degree of polymerisation (DPₙ), of 10-50, 10-40, 10-35, 10-30, 10-28, 10-25, 10-22, 10-20, 10-18, 10-16 or 10-14.

The alginate oligomer of the invention may have a degree of polymerisation (DP), or a number average degree of polymerisation (DPₙ), of 11-50, 11-40, 11-35, 11-30, 11-28, 11-25, 11-22, 11-20, 11-18, 11-16 or 11-14.

The alginate oligomer of the invention may have a degree of polymerisation (DP), or a number average degree of polymerisation (DPₙ), of 12-50, 12-40, 12-35, 12-30, 12-28, 12-25, 12-22, 12-20, 12-18, 12-16 or 12-14.

The alginate oligomer of the invention may have a degree of polymerisation (DP), or a number average degree of polymerisation (DPₙ), of 13-50, 13-40, 13-35, 13-30, 13-28, 13-25, 13-22, 13-20, 13-18, 13-16 or 13-14 (e.g. 13 or 14).

The alginate oligomer of the invention may have a degree of polymerisation (DP), or a number average degree of polymerisation (DPₙ), of 14-50, 14-40, 14-35, 14-30, 14-28, 14-25, 14-22, 14-20, 14-18, or 14-16.

The alginate oligomer of the invention may have a degree of polymerisation (DP), or a number average degree of polymerisation (DPₙ), of 15-50, 15-40, 15-35, 15-30, 15-28, 15-25, 15-22, 15-20, or 15-18.

The alginate oligomer of the invention may have a degree of polymerisation (DP), or a number average degree of polymerisation (DPₙ), of 18-50, 18-40, 18-35, 18-30, 18-28, 18-25, 18-22 or 18-20.

The alginate oligomer of the invention may have a degree of polymerisation (DP), or a number average degree of polymerisation (DPₙ), of 20-45, 20-40, 20-35, 20-30 or 20-25.

The alginate oligomer of the invention may have a degree of polymerisation (DP), or a number average degree of polymerisation (DPn), of 30-50, 30-45, 30-40 or 30-35.

Preferably the alginate oligomer of the invention is substantially free, preferably essentially free, of alginate oligomers having a degree of polymerisation outside of the ranges disclosed herein. This may be expressed in terms of the molecular weight distribution of the alginate oligomer of the invention, e.g. the percentage of each mole of the alginate oligomer being used in accordance with the invention which has a DP outside the relevant range. The molecular weight distribution is preferably such that no more than 10%, preferably no more than 9, 8, 7, 6, 5, 4, 3, 2, or 1% mole has a DP of three, two or one higher than the relevant upper limit for DPₙ. Likewise it is preferred that no more than 10%, preferably no more than 9, 8, 7, 6, 5, 4, 3, 2, or 1% mole has a DP below a number three, two or one smaller than the relevant lower limit for DPₙ.

Suitable alginate oligomers are described in WO2007/039754, WO2007/039760, WO 2008/125828, and WO2009/068841, the disclosures of which are explicitly cited herein.

Representative suitable alginate oligomers have a DPₙ in the range 5 to 30, a guluronate fraction (F_{G}) of at least 0.80, a mannuronate fraction (F_{M}) of no more than 0.20, and at least 95 mole% of DP no more than 25.

Further suitable alginate oligomers have a number average degree of polymerization in the range 7 to 15 (preferably 8 to 12), a guluronate fraction (F_{G}) of at least 0.85 (preferably at least 0.90), a mannuronate fraction (F_{M}) of no more than 0.15 (preferably no more than 0.10), and having at least 95% mole with a degree of polymerization less than 17 (preferably less than 14).

Further suitable alginate oligomers have a number average degree of polymerization in the range 5 to 18 (especially 7 to 15), a guluronate fraction (F_{G}) of at least 0.80 (preferably at least 0.85, especially at least 0.92), a mannuronate fraction (F_{M}) of no more than 0.20 (preferably no more than 0.15, especially no more than 0.08), and having at least 95% mole with a degree of polymerization less than 20 (preferably less than 17).

Further suitable alginate oligomers have a number average degree of polymerization in the range 5 to 18, a guluronate fraction (F_{G}) of at least 0.92, a mannuronate fraction (F_{M}) of no more than 0.08, and having at least 95% mole with a degree of polymerization less than 20.

Further suitable alginate oligomers have a number average degree of polymerization in the range 5 to 18 (preferably 7 to 15, more preferably 8 to 12, especially about 10), a guluronate fraction (F_{G}) of at least 0.80 (preferably at least 0.85, more preferably at least 0.90, especially at least 0.92, most especially at least 0.95), a mannuronate fraction (F_{M}) of no more than 0.20 (preferably no more than 0.15, more preferably no more than 0.10, especially no more than 0.08, most especially no more than 0.05), and having at least 95% mole with a degree of polymerization less than 20 (preferably less than 17, more preferably less than 14).

Further suitable alginate oligomers have a number average degree of polymerization in the range 7 to 15 (preferably 8 to 12), a guluronate fraction (F_{G}) of at least 0.92 (preferably at least 0.95), a mannuronate fraction (F_{M}) of no more than 0.08 (preferably no more than 0.05), and having at least 95% mole with a degree of polymerization less than 17 (preferably less than 14).

Further suitable alginate oligomers have a number average degree of polymerization in the range 5 to 18, a guluronate fraction (F_{G}) of at least 0.80, a mannuronate fraction (F_{M}) of no more than 0.20, and having at least 95% mole with a degree of polymerization less than 20.

Further suitable alginate oligomers have a number average degree of polymerization in the range 7 to 15, a guluronate fraction (F_{G}) of at least 0.85, a mannuronate fraction (F_{M}) of no more than 0.15, and having at least 95% mole with a degree of polymerization less than 17.

Further suitable alginate oligomers have a number average degree of polymerization in the range 7 to 15, a guluronate fraction (F_{G}) of at least 0.92, a mannuronate fraction (F_{M}) of no more than 0.08, and having at least 95% mole with a degree of polymerization less than 17.

Further suitable alginate oligomers have a number average degree of polymerization in the range 5 to 20, a guluronate fraction (F_{G}) of at least 0.85 and a mannuronate fraction (F_{M}) of no more than 0.15.

Further suitable alginate oligomers have a number average degree of polymerization in the range 5 to 20, a guluronate fraction (F_{G}) of 0.9-0.95 and a mannuronate fraction (F_{M}) of 0.05-0.1, which may be expressed as an alginate oligomer having 90-95% G residues and an average molecular weight of 2600 Da. Further suitable alginate oligomers have a number average degree of polymerization about 13 (e.g. 12, 13 or 14), a guluronate fraction (F_{G}) of at least about 0.80, 0.85, 0.87, 0.88, 0.90 or 0.93 (e.g. 0.92, 0.93 or 0.94) and a corresponding mannuronate fraction (F_{M}) of no more than about 0.20, 0.15, 0.13, 0.12, 0.10, or 0.07 (e.g. 0.08, 0.07 or 0.06).

Further suitable alginate oligomers have a number average degree of polymerization about 21 (e.g. 20, 21 or 22), a guluronate fraction (F_{G}) of at least about 0.80 (e.g. 0.85, 0.87, 0.88, 0.90, 0.92, 0.94 or 0.95) and a corresponding mannuronate fraction (F_{M}) of no more than about 0.20 (e.g. 0.15, 0.13, 0.12, 0.10, 0.08, 0.06, 0.05).

Further suitable alginate oligomers have a number average degree of polymerization about 6 (e.g. 5, 6 or 7), a guluronate fraction (F_{G}) of at least about 0.80 (e.g. 0.85, 0.87, 0.88, 0.90, 0.92, 0.94 or 0.95) and a corresponding mannuronate fraction (F_{M}) of no more than about 0.20 (e.g. 0.15, 0.13, 0.12, 0.10, 0.08, 0.06, 0.05).

It will thus be seen that a particular class of alginate oligomers favoured according to the present invention is alginate oligomers defined as so-called "high G" or "G-block" oligomers i.e. having a high content of G residues or G-blocks (e.g. wherein at least 70% of the monomer residues are G, preferably arranged in G-blocks). However, other types of alginate oligomer may also be used, including in particular "high M" or "M-block" oligomers or MG-block oligomers, as described further below. Accordingly, it is alginate oligomers with high proportions of a single monomer type, and with said monomers of this type being present predominantly in contiguous sequences of that monomer type, that represent oligomers that are particularly preferred, e.g. oligomers wherein at least 70% of the monomer residues in the oligomer are G residues linked 1-4 to another G-residue, or more preferably at least 75%, and most preferably at least 80, 85, 90, 92, 93, 94, 95, 96, 97, 98, 99% of the monomers residues of the oligomer are G residues linked 1-4 to another G residue. This 1-4 linkage of two G residues can be alternatively expressed as a guluronic unit bound to an adjacent guluronic unit.

In a further embodiment at least, or more particularly more than, 50% of the monomer residues of the alginate oligomer may be M residues (i.e. mannuronate or mannuronic acid). In other words the alginate oligomer will contain at least or alternatively more than 50% mannuronate (or mannuronic acid) residues. Specific embodiments thus include alginate oligomers with (e.g. containing) 50 to 70% M (mannuronate) residues or e.g. 70 to 100% M (mannuronate) residues. Further specific embodiments also include oligomers containing 71 to 85% M residues or 85 to 100% M residues. Thus, a representative alginate oligomer for use according to this embodiment of the present invention will contain more than 70% M residues (i.e. more than 70% of the monomer residues of the alginate oligomer will be M residues).

In other embodiments at least 50% or 60%, more particularly at least 70% or 75%, even more particularly at least 80, 85, 90, 95 or 99% of the monomer residues are mannuronate. In one embodiment the alginate oligomer may be an oligomannuronate (i.e. a homooligomer of M, or 100% M).

In a further embodiment, the above described alginates of the invention have a primary structure wherein the majority of the M residues are in so called M-blocks. In this embodiment preferably at least 50%, more preferably at least 70 or 75%, and most preferably at least 80, 85, 90 or 95% of the M residues are in M-blocks. An M block is a contiguous sequence of at least two M residues, preferably at least 3 contiguous M residues, more preferably at least 4 or 5 contiguous M residues, most preferably at least 7 contiguous M residues.

In particular, at least 90% of the M residues are linked 1-4 to another M residue. More particularly at least 95%, more preferably at least 98%, and most preferably at least 99% of the M residues of the alginate are linked 1-4 to another M residue.

Other preferred oligomers are alginate oligomers wherein at least 70% of the monomer residues in the oligomer are M residues linked 1-4 to another M-residue, or more preferably at least 75%, and most preferably at least 80, 85, 90, 92, 93, 94, 95, 96, 97, 98, 99% of the monomers residues of the oligomer are M residues linked 1-4 to another M residue. This 1-4 linkage of two M residues can be alternatively expressed as a mannuronic unit bound to an adjacent mannuronic unit.

In a still further embodiment, the alginate oligomers of the invention comprise a sequence of alternating M and G residues. A sequence of at least three, preferably at least four, alternating M and G residues represents an MG block. Preferably the alginate oligomers of the invention comprise an MG block. Expressed more specifically, an MG block is a sequence of at least three contiguous residues consisting of G and M residues and wherein each non-terminal (internal) G residue in the contiguous sequence is linked 1-4 and 4-1 to an M residue and each non-terminal (internal) M residue in the contiguous sequence is linked 1-4 and 4-1 to a G residue. Preferably the MG block is at least 5 or 6 contiguous residues, more preferably at least 7 or 8 contiguous residues.

In a further embodiment the minority uronate in the alginate oligomer (i.e. mannuronate or guluronate) is found predominantly in MG blocks. In this embodiment preferably at least 50%, more preferably at least 70 or 75% and most preferably at least 80, 85, 90 or 95% of the minority uronate monomers in the MG block alginate oligomer are present in MG blocks. In another embodiment the alginate oligomer is arranged such that at least 50%, at least 60%, at least 70%, at least 80%, at least 85%, at least 90%, at least 95%, at least 99%, e.g. 100% of the G and M residues in the oligomer are arranged in MG blocks.

Although at its broadest, the invention extends to embodiments wherein at least 1% but less than 100% of the monomer residues of the oligomer are G residues (i.e. guluronate or guluronic acid), more particularly, and as defined further below, at least 30% of the monomer residues are G residues. Thus, at its broadest the MG block containing alginate oligomer may contain at least 1%, but less than 100%, guluronate (or guluronic acid) residues, but generally the MG block containing alginate oligomer will contain at least 30% (or at least 35, 40 or 45% or 50% G) but less than 100% G. Specific embodiments thus include MG block containing alginate oligomers with (e.g. containing) 1 to 30% G (guluronate) residues, 30 to 70% G (guluronate) residues or 70 to 99% G (guluronate) residues. Thus, a representative MG block containing alginate oligomer for use according to the present invention may contain more than 30%, but less than 70%, G residues (i.e. more than 30%, but less than 70%, of the monomer residues of the MG block alginate oligomer will be G residues).

Preferably more than 30%, more particularly more than 35% or 40%, even more particularly more than 45, 50, 55, 60 or 65%, but in each case less than 70%, of the monomer residues of the MG block containing alginate oligomer are guluronate. Alternatively, less than 70%, more preferably less than 65% or 60%, even more preferably less than 55, 50, 45, 40 or 35%, but in each case more than 30% of the monomer residues of the MG block containing alginate oligomer are guluronate. Any range formed by any combination of these values may be chosen. Therefore for instance the MG block containing alginate oligomer can have e.g. between 35% and 65%, 40% and 60% or 45% and 55% G residues.

In another embodiment the MG block containing alginate oligomer may have approximately equal amounts of G and M residues (e.g. ratios between 65% G/35% M and 35% G/65% M, for instance 60% G/40% M and 40% G/60% M; 55% G/45% M and 45% G/55% M; 53% G/47% M and 47% G/53% M; 51% G/49% M and 49% G/51% M; e.g. about 50% G and about 50% M) and these residues are arranged predominantly, preferably entirely or as completely as possible, in an alternating MG pattern (e.g. at least 50% or at least 60, 70, 80, 85, 90 or 95% or 100% of the M and G residues are in an alternating MG sequence).

In certain embodiments the terminal uronic acid residues of the oligomers of use in the invention do not have a double bond, especially a double bond situated between the C₄ and C₅ atom. Such oligomers may be described as having saturated terminal uronic acid residues. The skilled man would be able to prepare oligomers with saturated terminal uronic acid residues without undue burden. This may be through the use of production techniques which yield such oligomers, or by converting (saturating) oligomers produced by processes that yield oligomers with unsaturated terminal uronic acid residues.

The alginate oligomer will typically carry a charge and so counter ions for the alginate oligomer may be any physiologically tolerable ion, especially those commonly used for charged drug substances, e.g. sodium, potassium, ammonium, chloride, mesylate, meglumine, etc. Ions which promote alginate gelation e.g. group 2 metal ions may also be used.

While the alginate oligomer may be a synthetic material generated from the polymerisation of appropriate numbers of guluronate and mannuronate residues, the alginate oligomers of use in the invention may conveniently be obtained, produced or derived from natural sources such as those mentioned above, namely natural alginate source materials.

Polysaccharide to oligosaccharide cleavage to produce the alginate oligomer useable according to the present invention may be performed using conventional polysaccharide lysis techniques such as enzymatic digestion and acid hydrolysis. In one favoured embodiment acid hydrolysis is used to prepare the alginate oligomers on the invention. In other embodiments enzymatic digestion is used with an additional processing step(s) to saturate the terminal uronic acids in the oligomers.

Oligomers may then be separated from the polysaccharide breakdown products chromatographically using an ion exchange resin or by fractionated precipitation or solubilisation or filtration. US 6,121,441 and WO 2008/125828, which are cited herein, describe a process suitable for preparing the alginate oligomers of use in the invention. Further information and discussion can be found in for example in "Handbooks of Hydrocolloids", Ed. Phillips and Williams, CRC, Boca Raton, Florida, USA, 2000, which textbook is explicitly incorporated by reference herein in its entirety.

The alginate oligomers may also be chemically modified, including but not limited to modification to add charged groups (such as carboxylated or carboxymethylated glycans) and alginate oligomers modified to alter flexibility (e.g. by periodate oxidation).

Alginate oligomers (for example oligoguluronic acids) suitable for use according to the invention may conveniently be produced by acid hydrolysis of alginic acid from, but not limited to, *Laminaria hyperbora* and *Lessonia nigrescens*, dissolution at neutral pH, addition of mineral acid reduce the pH to 3.4 to precipitate the alginate oligomer (oligoguluronic acid), washing with weak acid, resuspension at neutral pH and freeze drying.

The alginates for production of alginate oligomers of the invention can also be obtained directly from suitable bacterial sources e.g. *Pseudomonas aeruginosa* or *Azotobacter vinelandii.*

In embodiments where alginate oligomers which have primary structures in which the majority of the G residues are arranged in G-blocks rather than as single residues are required, algal sources are expected to be most suitable on account of the fact that the alginates produced in these organisms tend to have these structures. The bacterial sources may be more suitable for obtaining alginate oligomers of different structures.

The molecular apparatus involved in alginate biosynthesis in *Pseudomonas fluorescens* and *Azotobacter vinelandii* has been cloned and characterised (WO 94/09124; Ertesvåg, H., et al, Metabolic Engineering, 1999, Vol 1, 262-269; WO 2004/011628; Gimmestad, M., *et al (supra)*; Remminghorst and Rehm, Biotechnology Letters, 2006, Vol 28, 1701-1712; Gimmestad, M. et al, Journal of Bacteriology, 2006, Vol 188(15), 5551-5560) and alginates of tailored primary structures can be readily obtained by manipulating these systems.

The G content of alginates (for example an algal source material) can be increased by epimerisation, for example with mannuronan C-5 epimerases from A. *vinelandii* or other epimerase enzymes. Thus, for example *in vitro* epimerisation may be carried out with isolated epimerases from *Pseudomonas* or *Azotobacter,* e.g. AlgG from *Pseudomonas fluorescens* or *Azotobacter vinelandii* or the AlgE enzymes (AlgE1 to AlgE7) from *Azotobacter vinelandii.* The use of epimerases from other organisms that have the capability of producing alginate, particularly algae, is also specifically contemplated. The *in vitro* epimerisation of low G alginates with *Azotobacter vinelandii* AlgE epimerases is described in detail in Ertesvåg *et al (supra)* and Strugala et al (Gums and Stabilisers for the Food Industry, 2004, 12, The Royal Society of Chemistry, 84 - 94).

To obtain G-block containing alginates or alginate oligomers, epimerisation with one or more *Azotobacter vinelandii* AlgE epimerases other than AlgE4 is preferred as these enzymes are capable of producing G block structures. On the other hand AlgE4 epimerase can be used to create alginates or alginate oligomers with alternating stretches of M/G sequence or primary structures containing single G residue as it has been found that this enzyme seems preferentially to epimerise individual M residues so as to produce single G residues linked to M residues rather than producing G blocks. Particular primary structures can be obtained by using different combinations of these enzymes.

Mutated versions of these enzymes or homologues from other organisms are also specifically contemplated as of use. WO 94/09124 describes recombinant or modified mannuronan C-5 epimerase enzymes (AlgE enzymes) for example encoded by epimerase sequences in which the DNA sequences encoding the different domains or modules of the epimerases have been shuffled or deleted and recombined. Alternatively, mutants of naturally occurring epimerase enzymes, (AlgG or AlgE) may be used, obtained for example by site directed or random mutagenesis of the AlgG or AlgE genes.

A different approach is to create *Pseudomonas* and *Azotobacter* organisms that are mutated in some or all of their epimerase genes in such a way that those mutants produce alginates of the required structure for subsequent alginate oligomer production, or even alginate oligomers of the required structure and size (or molecular weight). The generation of a number of *Pseudomonas fluorescens* organisms with mutated AlgG genes is described in detail in WO 2004/011628 and Gimmestad, M., *et al*, 2003 (*supra*). The generation of a number of *Azotobacter vinelandii* organisms with mutated AlgE genes is disclosed in Gimmestad, M., *et al*, 2006 (*supra*).

A further approach is to delete or inactivate the endogenous epimerase genes from an *Azotobacter* or a *Pseudomonas* organism and then to introduce one or more exogenous epimerase genes, which may or may not be mutated (i.e. may be wild-type or modified) and the expression of which may be controlled, for example by the use of inducible or other "controllable promoters". By selecting appropriate combinations of genes, alginates of predetermined primary structure can be produced.

A still further approach would be to introduce some or all of the alginate biosynthesis machinery of *Pseudomonas* and/or *Azotobacter* into a non-alginate producing organism (e.g. *E. coli*) and to induce the production of alginate from these genetically modified organisms.

When these culture-based systems are used, the primary structure of the alginate or alginate oligomer products can be influenced by the culture conditions.

It is well within the capabilities of the skilled man to adjust culture parameters such as temperature, osmolarity, nutrient levels/sources and atmospheric parameters in order to manipulate the primary structure of the alginates produced by a particular organism.

References to "G residues/G" and "M residues/M" or to guluronic acid or mannuronic acid, or guluronate or mannuronate are to be read interchangeably as references to guluronic acid/guluronate and mannuronic acid/mannuronate (specifically α-L-guluronic acid/guluronate and β-D-mannuronic acid/mannuronate), and further include derivatives thereof in which one or more available side chains or groups have been modified without resulting in a capacity to widen the spectrum of a bacitacin's therapeutically useful activity against bacteria species to include Gram negative bacteria which is substantially lower than that of the unmodified oligomer. Common saccharide modifying groups would include acetyl, sulphate, amino, deoxy, alcohol, aldehyde, ketone, ester and anhydro groups. The alginate oligomers may also be chemically modified to add charged groups (such as carboxylated or carboxymethylated glycans), and to alter flexibility (e.g. by periodate oxidation). The skilled man would be aware of still further chemical modifications that can be made to the monosaccharide subunits of oligosaccharides and these can be applied to the alginate oligomers of use in the invention.

The direct covalent bond between the alginate oligomer and the bacitracin - class antibiotic is a covalent bond formed by an atom of the alginate oligomer and an atom of the bacitracin-class antibiotic. The atoms contributing to the bond may together or independently be carbon, oxygen, sulphur, nitrogen and/or phosphorous. The bond may be single, double or triple. In certain embodiments the bond is part of an organic functional group. The skilled person would be entirely familiar with the options available for suitable organic functional groups which could act as linkers between the alginate oligomer and the bacitracin-class antibiotics. Non-limiting examples thereof may include ester, carbonate ester, orthoester, ketone, ketal, hemiketal, ketene, ether, acetal, hemiacteal, peroxy, methylenedioxy, carbamate, amide, amine, amine oxide, hydroxamic acid, imine, imide, imidate, azide, azo, oxime, carbodiimide, carbazone, hydrozone, sulfide, disulfide, sulfinyl, sulfonyl, carbonothioyl, thioamide, thioester, thioether, thioketone, thioketal, sulphonate ester, dithiocarbamate, semicarbazone, phosphine or phosphodiester functional groups. As shown in the Examples, the formation of amide and ester bonds may be convenient and advantageous.

The covalent molecular linker may be any molecule, typically an organic molecule, or part thereof, which has a structure formed from covalently bonded atoms which is capable of bonding covalently with an alginate oligomer and a bacitracin-class antibiotic. Within the conjugate there will be a continuous series of covalently bonded atoms from the alginate oligomer to bacitracin-class antibiotic via the molecular linker. In preferred embodiments at least one of the covalent bonds in in said series is as defined above. The molecular linker may however further comprise non-covalent, e.g. ionic bonds, in parts of the molecule which are not contributing to the covalent linkage between the bacitracin-class antibiotic and the alginate oligomer.

The covalent molecular linker may be linear, circular or branched. In certain embodiments the molecular linker will have a molecular weight of equal to or less than 1500 Daltons, e.g. equal to or less than 1250, 1000, 900, 800, 700, 600, 500, 400, 300, 200 or 100 Daltons.

In certain embodiments at least one direct covalent bond between the alginate oligomer and the covalent molecular linker is as defined above. In certain embodiments at least one direct covalent bond between the bacitracin-class antibiotic and the covalent molecular linker is as defined above. Each bond may be the same or different. The covalent linker molecule may comprise at least one covalent bond as defined above, preferably in the part of that molecule which contributes to the continuous series of covalently bonded atoms from the alginate oligomer to bacitracin-class antibiotic via the molecular linker.

The covalent molecular linker may be or comprise an amino acid or a peptide, e.g. of equal to or fewer than 15 amino acid residues, e.g. of equal to or fewer than 12, 10, 8, 6, 5, 4, 3 or 2 amino acid residues. The amino acid may be and the peptide may comprise any of the amino acids described above. Specific examples of peptide linkers which may be used include but are not limited to peptides of Gly and/or Ser residues (e.g. (Gly)₂₋₈, (Ser)₂₋₈, (GGGGS)₁₋₃); (EAAAK)₁₋₃; A(EAAAK)₁₋₃A; Leu-Glu; (Xaa-Pro)₁₋₆ (e.g. (Glu-Pro)₁₋₆, (Lys-Pro)₁₋₆, (Ala-Pro)₁₋₆; VSQTSKLTR↓AETVFPDV (Factor Xla/Factor VIIa sensitive cleavage); PLG↓LWA (matrix metalloprotease-1 sensitive cleavage); RVL↓AEA (HIV-1 protease sensitive cleavage; EDVVCC↓SMSY (NS3 protease sensitive cleavage; GGIEGR↓GS (Factor Xa sensitive cleavage); TRHRQPR↓GWE (furin sensitive cleavage); AGNRVRR↓SVG (furin sensitive cleavage); GFLG↓ (Cathepsin B sensitive cleavage).

The covalent molecular linker may be or comprise a monosaccharide or an oligosaccharide other than guluronate or mannuronate or a polymer formed therefrom, e.g. a saccharide of equal to or fewer than 12 amino acid residues, e.g. equal to or fewer than 10, 8, 6, 5, 4, 3 or 2 amino acid residues. Thus the covalent molecular linker may be a monosaccharide, disaccharide or trisaccharide or sugar derivatives thereof such as aldonic and uronic acids, deoxy or amino sugars, sulfated sugars, and sugar alcohols. The monosaccharide or one or more of the monosaccharide residues of the disaccharide or trisaccharide may be a triose, a tetrose, a pentose, a hexose, a heptose, an octose, a nonose or a decose in pyranose or furanose form and/or L- or D- form where appropriate and/or sugar derivatives thereof. Pentose or hexose saccharides/residues are preferred, e.g. mannose (e.g. D-mannose), galactose (e.g. D- galactose), glucose (e.g. D-glucose), fructose, fucose (e.g. L-fucose), N-acetyl-glucosamine, N-acetylgalactosamine, rhamnose, galactosamine, glucosamine (e.g. D-glucosamine), galacturonic acid, glucuronic acid, N-acetylneuraminic acid, methyl D-mannopyranoside (mannoside), α-methyl-glucoside, galactoside, ribose, xylose, arabinose, saccharate, mannitol, sorbitol, inositol, glycerol and derivatives of these monomers. The disaccharide may be exemplified by acarviosin, allolactose, cellobiose, chitobiose, galactose-alpha-1,3-galactose, dentiobiose, isomalt, isomaltose, isomaltulose, kojibiose, lactitol, lactobionic acid, lactose, lactulose, laminaribiose, maltitol, maltose, mannobiose, melibiose, melibiulose, neohesperidose, nigerose, robinose, rutinose, sambubiose, sophorose, sucralfate, sucralose, sucrose, sucrose acetate isobutyrate, sucrose octaacetate, trehalose, truranose, xylobiose or derivatives of these disaccharides.

The covalent molecular linker may be or comprise a nucleotide or an oligonucleotide, i.e. a nucleic acid, e.g. a ribonucleotide or a deoxyribonucleotide.

The linker may also be or comprise a straight chain, branched or cyclic, substituted or unsubstituted, alkyl, alkenyl or alkynl group (typically C₂₋₈) or derivative thereof such as aminohexanoic acid or one of a range of commercially available PEG (polyethylene glycol) linkers.

Further examples of suitable covalent linker molecules include but are not limited to acetyl, succinyl, aconityl (*cis* or *trans*), glutaryl, methylsuccinyl, trimellityl cysteamine, penicillamine, N-(2-mercaptopropionyl)glycine, 2-mercaptopropionic acid, homocysteine, 3-mercaptopropionic acid and deamino-penicillamine groups.

In certain embodiments the covalent linker molecule may be a plurality of the molecules and/or groups described above.

In certain embodiments the direct covalent bond or the covalent linker molecule (more specifically a covalent bond within the linker molecule, a covalent bond between the linker molecule the alginate oligomer and/or a covalent bond between and the linker molecule and the bacitracin-class antibiotic) is selected for its ability to be lysed under conditions representative, or advantageously essentially unique to, a target site or location within a subject, e.g. conditions representative of a bacterial infection, the respiratory tract (especially the lower respiratory tract including the lungs, more particularly the lungs of a patient with cystic fibrosis) or wounds (in particular chronic wounds). In this way delivery of the bacitracin-class antibiotic may be made more selective for the target site.

In specific embodiments a covalent bond, a functional group containing said covalent bond or linker molecule may be selected which is sensitive to (labile at, degrades at, lyses at) a pH which is lower than normal physiological pH (pH 7.2), i.e. acidic pH, e.g. a pH of from about 3 to about 7, 6.5, 6, 5.5, 5, 4.5, 4, or 3.5. Sites or locations of inflammation, especially inflammation caused by infection typically have a pH in these ranges. Functional groups including esters, cis-aconityl, disulphides and hydrozones may be sensitive to lower pHs, i.e. may be described as acid labile.

In specific embodiments a covalent bond, functional group or linker molecule may be selected which is sensitive to reactive oxygen species. Sites or locations of inflammation, especially inflammation caused by infection typically have high levels of reactive oxygen species. Functional groups including thioketals and thioethers may be sensitive to reactive oxygen species

In further specific embodiments a covalent bond, functional group or linker molecule may be selected which is lysed by enzymes produced or secreted only at the target site or overproduced or oversecreted at the target site. This may include enzymes such as glycosidases, nucleases and peptidases, in particular those secreted by infecting bacteria and those secreted by inflammatory cells of the host, e.g. lysozyme, alginate lyase, DNasel, restriction endonucleases, neutrophil elastase, cathepsins, phospholipases and β-lactamases. It may however be advantageous to choose a covalent bond, functional group or linker molecule which is not lysed by enzymes capable of degrading the alginate oligomer or the bacitracin-class antibiotic and as such separation of the alginate oligomer from the bacitracin-class antibiotic will occur separately to the degradation of the alginate oligomer or bacitracin-class antibiotic.

In other embodiments the direct covalent bond or the covalent linker molecule may be selected for its stability under conditions representative, of advantageously essentially unique to, a target site or location within a subject, e.g. the location described above or locations or sites the conjugate may encounter en route to those locations and sites following administration and/or which the conjugate may encounter during its bodily distribution. An amide bond, a thioether bond or a Gly-Gly peptide linker may be, for example, suitable here.

In preferred embodiments the bacitracin-alginate oligomer conjugate consists of at least one alginate oligomer covalently bonded to a bacitracin-class antibiotic via an amide bond formed from a carboxyl group on the alginate and an amine group on the bacitracin molecule. Preferably the bacitracin-class antibiotic is selected from bacitracin A1, A2, B1, B2, B3, C, D1, D2, D3 and E and, more preferably, from bacitracin A (A1 and/or A2) and bacitracin B (B1 and/or B2). The alginate oligomer will preferably contain 2 to 100 monomer residues. The alginate oligomer may also have at least 70% G residues.

In preferred embodiments the bacitracin-alginate oligomer conjugate consists of at least one alginate oligomer covalently bonded to a bacitracin-class antibiotic via an ester bond formed from a carboxyl group on the alginate and hydroxyl group on the bacitracin molecule. Preferably the bacitracin-class antibiotic is selected from bacitracin A1, A2, B1, B2, B3, C, D1, D2, D3 and E and, more preferably, from bacitracin A (A1 and/or A2) and bacitracin B (B1 and/or B2). The alginate oligomer will preferably contain 2 to 100 monomer residues. The alginate oligomer may also have at least 70% G residues.

Multivalent arrangements are contemplated in which more than one alginate oligomer is covalently linked to the bacitracin-class antibiotic. The alginate oligomers may be the same or different and may be linked to the bacitracin-class antibiotic via the same type of covalent bond or covalent molecular linker. In other arrangements an alginate oligomer may be covalently linked to a plurality of bacitracin molecules in the manner described herein. The bacitracin molecules may be the same or different and may or may not be covalently linked to other alginate oligomers.

References to the bacitracin-alginate oligomer conjugates of the invention extends to pharmaceutically acceptable salts, solvates or hydrates thereof, diastereoisomers, tautomers, enantiomers, and active metabolites thereof. Suitable salts include acid addition salts from inorganic acids such as hydrochloric, sulphuric, phosphoric, nitric, carbonic, boric, sulfamic, and hydrobromic acids, or salts of pharmaceutically acceptable organic acids such as acetic, propionic, butyric, tartaric, maleic, hydroxymaleic, fumeric, citric, lactic, mucic, gluconic, benzoic, succinic, oxalic, phenylacetic, methanesulphonic, toluenesulphonic, benezenesulphonic, salicyclic, sulphanilic, aspartic, glutamic, edetic. stearic, palmitic, oleic, lauric, pantothenic, tannic, ascorbic, fendizoic, 4-4'-methylenebis-3-hydroxy-2-naphthoic acid, o-(p- hydroxybenzoyl)benzoic, 4-4'-dihydroxytriphenylmethane-2-carboxylic acid and valeric acids. Base salts include, but are not limited to, those formed with pharmaceutically acceptable cations, such as sodium, potassium, lithium, calcium, magnesium, ammonium and alkylammonium.

In a further aspect the invention provides a method for the preparation of a bacitracin-alginate oligomer conjugate of the invention, said method comprising
(ia) providing an alginate oligomer and a bacitracin-class antibiotic and forming a direct covalent bond between two molecular groups thereon; or
(ib) providing an alginate oligomer, a bacitracin-class antibiotic and a covalent molecular linker and forming a direct covalent bond between two molecular groups on the alginate oligomer and the linker molecule and forming a direct covalent bond between two molecular groups on the bacitracin-class antibiotic and the linker molecule; or
(ic) providing an alginate oligomer and a bacitracin-class antibiotic wherein one or both carry a covalent molecular linker molecule covalently bonded thereto and covalently linking the alginate oligomer to the bacitracin-class antibiotic via at least one of the linker molecules; and optionally (ii) separating at least a portion of the bacitracin-alginate oligomer conjugate from the reaction mixture.

In certain embodiments the invention provides a method for the preparation of a bacitracin-alginate oligomer conjugate of the invention, said method comprising
(i) providing an aqueous solution of an alginate oligomer having an available carboxyl group;
(ii) contacting said alginate solution with 1-ethyl-3-[3-dimethylaminopropyl]carbodiimide hydrochloride (EDC) in an amount and under conditions sufficient to activate at least one carboxyl group in the alginate oligomer;
(iii) optionally contacting said carboxyl activated alginate oligomer with sulfo N-hydroxysuccinimide (sulfo-NHS) in an amount and under conditions sufficient to form an amine-reactive sulfo-NHS ester;
(iv) contacting said carboxyl activated alginate oligomer of step (ii) or the amine-reactive sulfo-NHS ester of step (iii) with an bacitracin-class antibiotic having an available primary amine group in an amount and under conditions sufficient to form an amide bond between the alginate oligomer and the bacitracin-class antibiotic; and
(v) separating at least a portion of the bacitracin-alginate oligomer conjugate from the reaction mixture.

In certain embodiments the invention provides a method for the preparation of a bacitracin-alginate oligomer conjugate of the invention, said method comprising
(i) providing a solution of an alginate oligomer having an available carboxyl group, preferable an organic (e.g. DMF and/or DMSO) solution;
(ii) contacting said alginate solution with dicyclohexylcarbodiimide (DCC) in an amount and under conditions sufficient to form an O-acylisourea intermediate;
(iii) contacting said O-acylisourea intermediate with an bacitracin-class antibiotic having an available hydroxyl group and 4-N,N-dimethylaminopyridine (DMAP) in amounts and under conditions sufficient to form an ester bond between the alginate oligomer and the bacitracin-class antibiotic; and
(iv) separating at least a portion of the bacitracin-alginate oligomer conjugate from the reaction mixture;
   wherein steps (ii) and (iii) may be performed simultaneously.

As mentioned above and as shown in the Examples, bacitracin-alginate oligomer conjugates are a class of novel chemical entities having therapeutically useful antibacterial efficacy against both Gram negative and Gram positive bacteria, i.e. a spectrum of therapeutically useful activity which is wider than non-conjugated bacitracin-class antibiotics. This makes possible the effective treatment of bacterial infections (including Gram negative infections).

Thus, in a further aspect the invention provides a pharmaceutical composition comprising a bacitracin-alginate oligomer conjugate as defined herein and a pharmaceutically acceptable excipient, carrier or diluent. Suitable excipients, carriers or diluents are described and specific pharmaceutical compositions are detailed below.

The invention further relates to the use of the bacitracin-alginate oligomer conjugates as described herein and the pharmaceutical compositions comprising the same in the combat of bacterial infection, in particular Gram negative bacterial infection. The term "combat" as used herein includes both therapy and prophylaxis (i.e. the treatment or prevention of a bacterial infection).

Thus in this aspect the invention provides the bacitracin-alginate oligomer conjugates of the invention as described herein and pharmaceutical compositions comprising them for use in therapy, particularly for use in the treatment or prevention of a bacterial infection, preferably a Gram negative bacterial infection.

Specifically, in a further aspect the invention provides a method for the treatment or prevention of a bacterial infection, preferably a Gram negative bacterial infection, in a subject with, suspected to have, or at risk of, a bacterial infection, said method comprising administering to said subject an effective amount of a bacitracin-alginate oligomer conjugate of the invention as defined herein.

The invention further provides a bacitracin-alginate oligomer conjugate of the invention as defined herein, for use in the treatment or prevention of a bacterial infection, preferably a Gram negative bacterial infection, in a subject with, suspected to have, or at risk of, a bacterial infection.

An "effective", more particularly a "pharmaceutically effective", amount of the bacitracin-alginate oligomer conjugate is that amount of conjugate that provides a measurable treatment or prevention of the target bacterial infection, e.g. a Gram negative bacterial infection.

In the above described embodiments the primary physiological result to be achieved is the contact of the site of infection (in particular the bacteria which are present in the infected site or location, and which may include multiple sites or locations of infection in the body, including also a systemic infection) and/or a site (e.g. a surface) at which the infection may occur (or is at risk of occurring) with the bacitracin-alginate oligomer conjugate.

Expressed alternatively the invention further provides the use of an a bacitracin-alginate oligomer conjugate of the invention as defined herein for the manufacture of a medicament for use in the treatment or prevention of a bacterial infection, preferably a Gram negative bacterial infection, in a subject with, suspected to have, or at risk of, a bacterial infection.

The term "bacterial infection" (or "infected by" or "infected with" and the like) is used broadly herein to indicate that the subject may comprise, or contain, or carry, the bacteria in question, i.e. that the bacteria may simply be present in or on the subject, and this may include any site or location in or on the body of the subject. It is not necessary that the infection of the subject be manifest as a clinical disease (i.e. that the infection result in clinical symptoms in the subject), although this is of course encompassed. A subject who is suspected to be infected or who is at risk of infection may be a subject who has been exposed to the bacteria or to an infected subject, or a subject presenting with clinical signs or symptoms of infection (in the case of a suspected infection), or a subject who is susceptible to infection, whether generally (e.g. due to the clinical status of the subject) or particularly to the bacteria in question.

Also in accordance with certain aspects of the invention there may be a preceding step of identifying a subject as being a subject with, suspected to have, or at risk of, a bacterial infection, or a step of diagnosing a subject as a subject with, suspected to have, or at risk of, a bacterial infection. In particular, the bacterial infection may be of a type which is known to be untreatable, or at least difficult to treat, in usual clinical practice with a bacitracin-class antibiotic. In one embodiment the bacteria are identified as or suspected to be bacteria which are unresponsive to (i.e. sensitive to) a bacitracin-class antibiotic, at least at therapeutic doses. In certain embodiments the sensitivity/resistance of that infection (or more particularly the bacteria within the infection) to a bacitracin-class antibiotic may be determined.

Alternatively or in addition to the above described preceding step, in accordance with the invention there may be a following step in which the subject's clinical indicators of the bacterial infection are assessed and preferably compared to a corresponding assessment made prior to, or earlier in, said treatment in order to determine any changes therein.

The diagnosis and monitoring of bacterial infections based on readily observable physiological indicators is entirely routine for clinicians. Molecular biological and microbiological methods may also be used to more confirm diagnoses and to provide more information on the causative agents, e.g. taxonomic information, possible indications of virulence and their sensitivity to antibiotics.

The invention encompasses the use of a single bacitracin-alginate oligomer conjugate or a mixture (multiplicity/plurality; two or more) of different bacitracin-alginate oligomer conjugates. Such mixtures may comprise conjugates carrying different bacitracin-class antibiotics and the same alginate oligomer. Such mixtures may comprise conjugates carrying the same bacitracin-class antibiotic and different alginate oligomers. Such mixtures may comprise conjugates carrying different bacitracin-class antibiotics and different alginate oligomers..

The bacterial infection targeted according to the invention may comprise bacteria from any genera or species of bacteria. Examples of genera or species of bacteria include, but are not limited to, *Abiotrophia, Achromobacter, Acidaminococcus, Acidovorax, Acinetobacter, Actinobacillus, Actinobaculum, Actinomadura, Actinomyces, Aerococcus, Aeromonas, Afipia, Agrobacterium, Alcaligenes, Alloiococcus, Alteromonas, Amycolata, Amycolatopsis, Anaerobospirillum, Anaerorhabdus, Arachnia, Arcanobacterium, Arcobacter, Arthrobacter, Atopobium, Aureobacterium, Bacteroides, Balneatrix, Bartonella, Bergeyella, Bifidobacterium, Bilophila Branhamella, Borrelia, Bordetella, Brachyspira, Brevibacillus, Brevibacterium, Brevundimonas, Brucella, Burkholderia, Buttiauxella, Butyrivibrio, Calymmatobacterium, Campylobacter, Capnocytophaga, Cardiobacterium, Catonella, Cedecea, Cellulomonas, Centipeda, Chlamydia, Chlamydophila, Chromobacterium, Chyseobacterium, Chryseomonas, Citrobacter, Clostridium, Collinsella, Comamonas, Corynebacterium, Coxiella, Cryptobacterium, Delftia, Dermabacter, Dermatophilus, Desulfomonas, Desulfovibrio, Dialister, Dichelobacter, Dolosicoccus, Dolosigranulum, Edwardsiella, Eggerthella, Ehrlichia, Eikenella, Empedobacter, Enterobacter, Enterococcus, Erwinia, Erysipelothrix, Escherichia, Eubacterium, Ewingella, Exiguobacterium, Facklamia, Filifactor, Flavimonas, Flavobacterium, Francisella, Fusobacterium, Gardnerella, Globicatella, Gemella, Gordona, Haemophilus, Hafnia, Helicobacter, Helococcus, Holdemania, Ignavigranum, Johnsonella, Kingella, Klebsiella, Kocuria, Koserella, Kurthia, Kytococcus, Lactobacillus, Lactococcus, Lautropia, Leclercia, Legionella, Leminorella, Leptospira, Leptotrichia, Leuconostoc, Listeria, Listonella, Megasphaera, Methylobacterium, Microbacterium, Micrococcus, Mitsuokella, Mobiluncus, Moellerella, Moraxella, Morganella, Mycobacterium, Mycoplasma, Myroides, Neisseria, Nocardia, Nocardiopsis, Ochrobactrum, Oeskovia, Oligella, Orientia, Paenibacillus, Pantoea, Parachlamydia, Pasteurella, Pediococcus, Peptococcus, Peptostreptococcus, Photobacterium, Photorhabdus, Plesiomonas, Porphyrimonas, Prevotella, Propionibacterium, Proteus, Providencia, Pseudomonas, Pseudonocardia, Pseudoramibacter, Psychrobacter, Rahnella, Ralstonia, Rhodococcus, Rickettsia Rochalimaea Roseomonas, Rothia, Ruminococcus, Salmonella, Selenomonas, Serpulina, Serratia, Shewenella, Shigella, Simkania, Slackia, Sphingobacterium, Sphingomonas, Spirillum, Staphylococcus, Stenotrophomonas, Stomatococcus, Streptobacillus, Streptococcus, Streptomyces, Succinivibrio, Sutterella, Suttonella, Tatumella, Tissierella, Trabulsiella, Treponema, Tropheryma, Tsakamurella, Turicella, Ureaplasma, Vagococcus, Veillonella, Vibrio, Weeksella, Wolinella, Xanthomonas, Xenorhabdus, Yersinia, and Yokenella;* e.g. Gram-positive bacteria including *Staphylococcus aureus, Staphylococcus epidermidis, Staphylococcus equi, Streptococcus pyogenes, Streptococcus agalactiae, Listeria monocytogenes, Listeria ivanovii, Bacillus anthracis, B. subtilis, Nocardia asteroides, Actinomyces israelii, Propionibacterium acnes, Clostridium tetani, Clostridium perfringens, Clostridium botulinum, and Enterococcus species,* Gram-negative bacteria including *Pseudomonas aeruginosa, Vibrio cholerae, Actinobacillus pleuropneumoniae, Pasteurella haemolytica, Pasteurella multocida, Legionella pneumophila, Salmonella typhi, Brucella abortus, Coxiella burnetti, Escherichia coli, Neiserria meningitidis, Neiserria gonorrhea, Haemophilus influenzae, Haemophilus ducreyi, Yersinia pestis, Yersinia enterolitica, Escherichia hirae, Burkholderia cepacia, Burkholderia mallei, Burkholderia pseudomallei, Francisella tularensis, Bacteroides fragilis, Fusobascterium nucleatum, Cowdria ruminantium, Moraxella catarrhalis, Klebsiella pneumoniae, Proteus mirabilis, Enterobacter cloacae, Serratia marcescens, Helicobacter pylori, Salmonella enteritidis, Salmonella typhi and Acinetobacter baumannii, Acinetobacter Iwoffi, Providencia stuartii, Providencia rettgeri, Providencia alcalifaciens and Klebsiella oxytoca,* and Gram non-responsive bacteria including *Chlamydia trachomatis, Chlamydia psittaci* and mycobacteria such as M. *tuberculosis, M. bovis, M. typhimurium, M. bovis strain BCG, BCG substrains, M. avium, M. intracellulare, M. africanum, M. kansasii, M. marinum, M. ulcerans, M. avium subspecies paratuberculosis,*

Preferably the bacterial infection targeted according to the invention comprises bacteria selected from the following genera: *Achromobacter, Acinetobacter, Actinobacillus, Aeromonas, Agrobacterium, Alcaligenes, Alteromonas, Bacteroides, Bartonella, Borrelia, Bordetella, Brucella, Burkholderia, Campylobacter, Cardiobacterium, Chlamydia, Chlamydophila, Chromobacterium, Chyseobacterium, Chryseomonas, Citrobacter, Clostridium, Comamonas, Corynebacterium, Coxiella, Cryptobacterium, Edwardsiella, Eikenella, Enterobacter, Enterococcus, Erwinia, Kingella, Klebsiella, Lactobacillus, Lactococcus, Legionella, Leptospira, Leptotrichia, Leuconostoc, Listeria, Listonella, Mobiluncus, Moraxella, Morganella, Mycobacterium, Mycoplasma, Neisseria, Nocardia, Nocardiopsis, Pantoea, Parachlamydia, Pasteurella, Peptococcus, Peptostreptococcus, Prevotella, Propionibacterium, Proteus, Providencia, Pseudomonas, Ralstonia, Rickettsia, Salmonella, Shewenella, Shigella, Sphingobacterium, Sphingomonas, Staphylococcus, Stenotrophomonas, Streptobacillus, Streptococcus, Streptomyces, Treponem and Yersinia*

Thus, the invention may be used against Gram positive or Gram negative bacteria, or indeed Gram-indeterminate bacteria, but use against Gram-negative bacteria, for instance those particularised above, is preferred. These would include bacteria from the genera or species *Achromobacter, Acidaminococcus, Acidovorax, Acinetobacter, Actinobacillus, Aeromonas, Afipia, Agrobacterium, Alcaligenes, Alteromonas, Anaerobospirillum, Anaerorhabdus, Arcobacter, Bacteroides, Balneatrix, Bartonella, Bergeyella, Bilophila, Branhamella, Borrelia, Bordetella, Brachyspira, Brevibacillus, Brevundimonas, Brucella, Burkholderia, Buttiauxella, Calymmatobacterium, Campylobacter, Capnocytophaga, Cardiobacterium, Catonella, Cedecea, Centipeda, Chlamydia, Chlamydophila, Chromobacterium, Chyseobacterium, Chryseomonas, Citrobacter, Comamonas, Coxiella, Delftia, Desulfomonas, Desulfovibrio, Dialister, Dichelobacter, Edwardsiella, Ehrlichia, Eikenella, Empedobacter, Enterobacter, Erwinia, Escherichia, Ewingella, Flavimonas, Flavobacterium, Francisella, Fusobacterium, Gardnerella, Haemophilus, Hafnia, Helicobacter, Johnsonella, Kingella, Klebsiella, Koserella, Lautropia, Leclercia, Legionella, Leminorella, Leptospira, Leptotrichia, Listonella, Megasphaera, Methylobacterium, Mitsuokella, Moellerella, Moraxella, Morganella, Mycoplasma, Myroides, Neisseria, Ochrobactrum, Oligella, Orientia, Pantoea, Parachlamydia, Pasteurella, Photobacterium, Photorhabdus, Plesiomonas, Porphyrimonas, Prevotella, Proteus, Providencia, Pseudomonas, Psychrobacter, Rahnella, Ralstonia, Rickettsia, Rochalimaea, Roseomonas, Salmonella, Selenomonas, Serpulina, Serratia, Shewenella, Shigella, Simkania, Sphingobacterium, Sphingomonas, Spirillum, Stenotrophomonas, Streptobacillus, Succinivibrio, Sutterella, Suttonella, Tatumella, Tissierella, Trabulsiella, Treponema, Ureaplasma, Veillonella, Vibrio, Weeksella, Wolinella, Xanthomonas, Xenorhabdus, Yersinia, and Yokenella;* e.g. *Pseudomonas aeruginosa, Vibrio cholerae, Actinobacillus pleuropneumoniae, Pasteurella haemolytica, Pasteurella multocida, Legionella pneumophila, Salmonella typhi, Brucella abortus, Coxiella burnetti, Escherichia coli, Neiserria meningitidis, Neiserria gonorrhea, Haemophilus influenzae, Haemophilus ducreyi, Yersinia pestis, Yersinia enterolitica, Escherichia hirae, Burkholderia cepacia, Burkholderia mallei, Burkholderia pseudomallei, Francisella tularensis, Bacteroides fragilis, Fusobascterium nucleatum, Cowdria ruminantium, Moraxella catarrhalis, Klebsiella pneumoniae, Proteus mirabilis, Enterobacter cloacae, Serratia marcescens, Helicobacter pylori, Salmonella enteritidis, Salmonella typhi and Acinetobacter baumannii, Acinetobacter Iwoffi, Providencia stuartii, Providencia rettgeri, Providencia alcalifaciens and Klebsiella oxytoca.*

Preferably the Gram negative bacterium targeted according to the invention is selected from the following genera: *Achromobacter, Acinetobacter, Actinobacillus, Aeromonas, Agrobacterium, Alcaligenes, Alteromonas, Bacteroides, Bartonella, Borrelia, Bordetella, Brucella, Burkholderia, Campylobacter, Cardiobacterium, Chlamydia, Chlamydophila, Chromobacterium, Chyseobacterium, Chryseomonas, Citrobacter, Comamonas, Coxiella, Edwardsiella, Eikenella, Enterobacter, Erwinia, Kingella, Klebsiella, Legionella, Leptospira, Leptotrichia, Listonella, Moraxella, Morganella, Mycoplasma, Neisseria, Pantoea, Parachlamydia, Pasteurella, Prevotella, Proteus, Providencia, Pseudomonas, Ralstonia, Rickettsia, Salmonella, Shewenella, Shigella, Sphingobacterium, Sphingomonas, Stenotrophomonas, Streptobacillus, Treponem and Yersinia.*

Within the Gram-negative bacteria the *Enterobacteriaceae* and the Gram-negative bacteria non-fermenting bacteria are of particular note.

*Enterobacteriaceae* include, but are not limited to, bacteria from the genera *Alishewanella, Alterococcus, Aquamonas, Aranicola, Azotivirga, Brenneria, Budvicia, Buttiauxella, Cedecea, Citrobacter, Cronobacter, Dickeya, Edwardsiella, Enterobacter, Erwinia, Escherichia, Ewingella, Grimontella, Hafnia, Klebsiella, Kluyvera, Leclercia, Leminorella, Moellerella, Morganella, Obesumbacterium, Pantoea, Pectobacterium, Phlomobacter, Photorhabdus, Plesiomonas, Pragia, Proteus, Providencia, Rahnella, Raoultella, Salmonella, Samsonia, Serratia, Shigella, Sodalis, Tatumella, Trabulsiella, Wigglesworthia, Xenorhabdus, Yersinia, Yokenella.* Preferred genera of *Enterobacteriaceae* include *Escherichia, Klebsiella, Salmonella, Shigella, Yersinia* and *Providencia.*

Non-fermenting Gram-negative bacteria include, but are not limited to, bacteria from the genera *Pseudomonas, Acinetobacter, Stenotrophomonas and Burkholderia, Achromobacter, Algaligenes, Bordetella, Brevundimonas, Comamonas, Elizabethkingia* (formerly *Chryseobacterium), Methylobacterium, Moraxella, Ochrobactrum, Oligella, Psychrobacter, Ralstonia, Roseomonas, Shewanella, Sphingobacterium,* e.g. *Pseudomonas aeruginosa, Acinetobacter baumannii, Stenotrophomonas maltophilia,* and *Burkholderia spp..*

Preferably the bacteria may be selected from the genera *Pseudomonas, Acinetobacter, Stenotrophomonas, Burkholderia, Escherichia, Klebsiella, Providencia,* e.g. *Pseudomonas aeruginosa, Acinetobacter baumannii, Stenotrophomonas maltophilia, Burkholderia spp, E. coli, Klebsiella pneumoniae and Burkholderia cepacia, Burkholderia mallei, Burkholderia pseudomallei, Acinetobacter Iwoffi, Providencia stuartii, Providencia rettgeri, Providencia alcalifaciens, Klebsiella oxytoca, Pseudomonas anguilliseptica, Pseudomonas oryzihabitans, Pseudomonas plecoglossicida* and *Pseudomonas luteola. More* preferably the bacteria may be selected from the genera *Pseudomonas, Acinetobacter, Escherichia* and *Klebsiella,* e.g. *Pseudomonas aeruginosa, Acinetobacter baumannii, E. coli,* and *Klebsiella pneumoniae.*

In certain aspects, the infection is a nosocomial infection, an infection in the respiratory tract of patients, e.g. in patients suffering from cystic fibrosis, chronic obstructive pulmonary disease, congestive obstructive airway disease / congestive obstructive airway pneumonia (COAD/COAP), pneumonia, emphysema, bronchitis or sinusitis; an infection in a wound, particularly a chronic wound (including burns), a device related infection associated with implantable or prosthetic medical devices e.g. prosthetic valve endocarditis or an infection of a line or a catheter or an artificial joints or a tissue replacements or an endotracheal or tracheotomy tube. Examples of the types of bacteria which commonly cause such infections include *Pseudomonas aeruginosa, Acinetobacter baumannii, Stenotrophomonas maltophilia, Burkholderia spp (e.g. B. cepacia), E. coli, Klebsiella pneumoniae, Staphylococcus aureus,* Methicillin Resistant *Staphylococcus aureus* (MRSA), *Clostridium difficile, Mycobacterium tuberculosis, Enterococcus and Vancomycin-Resistant Enterococcus* and *Providencia stuartii.* Other infections of importance in accordance with the invention include infections by Bartonella (e.g. *Bartonella hensela),* mycobacteria *(e.g. Mycobacterium avium* Complex (MAC), *M*. *kansasii, M. marinum, M. ulcerans, M. xenopi),* Haemophilus influenzae type b (Hib) or Legionella *(e.g. Legionella pneumophila;* Legionnaire's disease), leprosy (*M*. *leprae*), human granulocytic anaplasmosis (*Anaplasma phagocytophilum*), brucellosis (*Brucella melitensis),* meningococcal disease (*Neisseria meningitides*) and anthrax *(Bacillus anthracis).*

The bacteria may be multidrug resistant, e.g. bacteria which are resistant to antibiotics from at least 3, or at least 4, 5, 6, 7, 8, 9 or 10 antibiotic classes, e.g. the aminoglycosides (e.g. amikacin, gentamicin, kanamycin, capreomycin, neomycin, netilmicin, streptomycin, tobramycin); the β-lactams (e.g. the carbecephems (e.g. loracarbef); the 1st generation cephalosporins (e.g. cefadroxil, cefazolin, cephalexin); 2nd generation cephalosporins (e.g. cefaclor, cefamandole, cephalexin, cefoxitin, cefprozil, cefuroxime); 3rd generation cephalosporins (e.g. cefixime, cefdinir, cefditoren, cefoperazone, cefotaxime, cefpodoxime, ceftazidime, ceftibuten, ceftizoxime, ceftriaxone); 4th generation cephalosporins (e.g. cefepime); the monobactams (e.g. aztreonam)); the macrolides (e.g. azithromycin, clarithromycin, dirithromycin, erythromycin, troleandomycin); the monobactams (e.g. aztreonam); the penicillins (e.g. amoxicillin, ampicillin, carbenicillin, cloxacillin, dicloxacillin, nafcillin, oxacillin, penicillin G, penicillin V, piperacillin, ticarcillin); the polypeptide antibiotics (e.g. bacitracin, colistin and polymyxin B, but in certain embodiments not bacitracin); the quinolones (e.g. ciprofloxacin, enoxacin, gatifloxacin, levofloxacin, lomefloxacin, moxifloxacin, norfloxacin, ofloxacin, trovafloxacin); the sulfonamides (e.g. mafenide, sulfacetamide, sulfamethizole, sulfasalazine, sulfisoxazole, trimethoprim- sulfamethoxazole); the tetracyclines (e.g. demeclocycline, doxycycline, minocycline, oxytetracycline, tetracycline); the glycylcyclines (e.g. tigecycline); the carbapenems (e.g. imipenem, meropenem, ertapenem, doripenem, panipenem/betamipron, biapenem, PZ-601); and other antibiotics including chloramphenicol; clindamycin, ethambutol; fosfomycin; isoniazid; linezolid; metronidazole; nitrofurantoin; pyrazinamide; quinupristin/dalfopristin; spectinomycin; fosfomycin and vancomycin.

Susceptibility, that is sensitivity, (and conversely resistance and tolerance) to an antibiotic can be measured in any convenient way, e.g. with dilution susceptibility tests and/or disk diffusion tests. Preferably the susceptibility of a bacterial strain to an antibiotic is expressed in terms of the Minimum Inhibitory Concentration (MIC) of that antibiotic for that microorganism (Jorgensen et al., Manual of Clinical Microbiology, 7th ed. Washington, D.C: American Society for Microbiology, 1999; 1526-43), i.e. that concentration of antibiotic that completely inhibits growth of that bacterial strain.

The skilled man would appreciate that the extent of the difference in tolerance/susceptibility sufficient to constitute resistance will vary depending on the antibiotic and bacterial strain under test and the test used. Many regulatory bodies, e.g. the European Committee on Antimicrobial Susceptibility Testing (EUCAST) set so called "breakpoints" for specific antimicrobials and microorganisms which are discriminatory antimicrobial concentrations used in the interpretation of results of susceptibility testing to define isolates as susceptible, intermediate or resistant to the antimicrobial agent under test. The skilled person can utilise such information to ascertain whether or not the bacterial infection being treated by the invention is resistant to the antibiotic in question under these definitions.

In certain embodiments the target infection will be bacteria in a biofilm. However, in other embodiments the bacterium will not be in a biofilm.(e.g. will be growing planktonically). Put differently, the bacterium will be, or will not be, in a biofilm mode of growth; or will be, or will not be, in a non-biofilm mode of growth.

By "biofilm" it is meant a community of microorganisms characterized by a predominance of sessile cells that are attached to a substratum or interface or to each other (some motile cells may also be present) and that are embedded in a matrix of extracellular polymers (more specifically extracellular polymers that they have produced) characterised in that the microorganisms of this colony exhibit an altered phenotype with respect to growth rate and gene transcription (for example as compared to their "non-biofilm" or free-floating or planktonic counterparts). By "in a biofilm" it is meant that the bacterium targeted by the method of the invention is within (completely or in part), on or associated with the polymer matrix of a biofilm. Viewed differently, bacteria that are "not in a biofilm" are organisms that are either in isolation, e.g. planktonic, or if in an aggregation of a plurality of organisms, that aggregation is unorganised and/or is devoid of the matrix characteristic of a biofilm. In each case, the individual bacteria do not exhibit an altered phenotype that is observed in their biofilm dwelling counterparts.

In certain embodiments the bacterial infection does not contain bacteria from the genus Acinetobacter. In certain embodiments the bacterial infection to be treated or prevented in accordance with the invention does not contain bacteria which are multidrug resistant.

In particular embodiments the invention may provide for the treatment or prevention of respiratory infections or conditions associated therewith (e.g. cystic fibrosis, pneumonia, COPD, COAD, COAP, bronchitis, sinusitis, an infection in a chronic wound (including burns), a device related infection associated with implantable or prosthetic medical devices, bacteraemia, septicaemia, septic shock, or sepsis.

In one preferred embodiment the bacterial infection is a respiratory infection in a subject suffering from an underlying respiratory disorder or condition, including notably CF, COPD/COAD, or asthma.

"Treatment" when used in relation to the treatment of a bacterial infection/medical condition in a subject in accordance with the invention is used broadly herein to include any therapeutic effect, i.e. any beneficial effect in relation to the infection or on the condition. Thus, not only included is eradication or elimination of the infection, or cure of the subject or infection, but also an improvement in the infection or condition of the subject. Thus included for example, is an improvement in any symptom or sign of the infection or condition, or in any clinically accepted indicator of the infection/condition (for example a decrease in wound size or an acceleration of healing time). Treatment thus includes both curative and palliative therapy, e.g. of a pre-existing or diagnosed infection/condition, i.e. a reactionary treatment.

"Prevention" as used herein refers to any prophylactic or preventative effect. It thus includes delaying, limiting, reducing or preventing the infection/condition or the onset of the infection/condition, or one or more symptoms or indications thereof, for example relative to the infection/condition or symptom or indication prior to the prophylactic treatment. Prophylaxis thus explicitly includes both absolute prevention of occurrence or development of the infection/condition, or symptom or indication thereof, and any delay in the onset or development of the infection/condition or symptom or indication thereof, or reduction or limitation of the development or progression of the infection/condition or symptom or indication thereof.

The subject may be any human or non-human animal subject, but more particularly may be a human or a non-human vertebrate, e.g. a non-human mammal, bird, amphibian, fish or reptile. In a preferred embodiment the subject is a mammalian subject. The animal may be a livestock or a domestic animal or an animal of commercial value, including laboratory animals or an animal in a zoo or game park. Representative animals therefore include dogs, cats, rabbits, mice, guinea pigs, hamsters, horses, pigs, sheep, goats and cows. Veterinary uses of the invention are thus covered. The subject may be viewed as a patient. Preferably the subject is a human. In some embodiments the subject is not a ruminant mammal.

The term "in a subject" is used broadly herein to include sites or locations inside a subject or on a subject, e.g. an external body surface, and may include in particular infection of a medical device e.g. an implanted or "in-dwelling" medical device. The term "in a patient" should be interpreted consistently with this.

The location of the infection may therefore be a surface in the oral cavity (e.g. teeth, gingiva, gingival crevice, periodontal pocket), the reproductive tract (e.g. cervix, uterus, fallopian tubes), the peritoneum, middle ear, prostate, the urinary tract, vascular intima, the eye, i.e. ocular tissue (e.g. the conjunctiva, corneal tissue, lachrymal duct, lachrymal gland, eyelid) the respiratory tract, lung tissue (e.g. bronchial and alveolial), heart valves, the gastrointestinal tract, skin, scalp, nails and the interior of wounds, particularly chronic wounds and surgical wounds, which may be topical or internal wounds. Other surfaces include the exterior of organs, particularly those undergoing transplantation, for example, heart, lungs, kidney, liver, heart valve, pancreas, intestine, corneal tissue, arterial and venous grafts and skin.

The infection may therefore also be present in body fluids (e.g. blood, plasma, serum, cerebrospinal fluid, GI tract contents, semen, sputum and other pulmonary secretions) and tissues (e.g. adrenal, hepatic, renal, pancreatic, pituitary, thyroid, immune, ovarian, testicular, prostate, endometrial, ocular, mammary, adipose, epithelial, endothelial, neural, muscle, pulmonary, epidermis, osseous).

The infection may further be found on any "in-dwelling" medical or surgical equipment or devices. This may include any kind of line, including catheters (e.g. central venous and urinary catheters), prosthetic devices e.g., heart valves, artificial joints, false teeth, dental crowns, dental caps and soft tissue implants (e.g. breast, buttock and lip implants). Any kind of implantable medical device is included (e.g. stents, intrauterine devices, pacemakers, intubation tubes (e.g. endotracheal or tracheostomy tubes), prostheses or prosthetic devices, lines or catheters). An "in-dwelling" medical device may include a device in which any part of it is contained within the body, i.e. the device may be wholly or partly in-dwelling.

The infection may be acute, or alternatively chronic, e.g. an infection that has persisted for at least 5 or at least 10 days, particularly at least 20 days, more particularly at least 30 days, most particularly at least 40 days.

A bacterial infection can occur in any subject but some subjects will be more susceptible to infection that others. Subjects who are susceptible to bacterial infection include, but are not limited to, subjects whose epithelial and/or endothelial barrier is weakened or compromised, subjects whose secretion-based defences to microbial infection have been abrogated, disrupted, weakened or undermined, and subjects who are immunocompromised, immunodeficient or immunosuppressed (i.e. a subject in whom any part of the immune system is not working normally, or is working sub-normally, in other words in whom any part of the immune response, or an immune activity is reduced or impaired, whether due to disease or clinical intervention or other treatment, or in any way).

Representative examples of subjects who are susceptible to bacterial infection include, but are not limited to, subjects with a pre-established infection (e.g. with bacteria, viruses, fungi or parasites such as protozoa), especially subjects with HIV, subjects with bacteraemia, sepsis and subjects with septic shock; subjects with immunodeficiency, e.g. subjects preparing for, undergoing or recovering from chemotherapy and/or radiotherapy, organ (e.g. bone marrow, liver, lung, heart, heart valve, kidney, etc.) transplant subjects (including autograft, allograft and xenograft patients); subjects with AIDS; subjects resident in a healthcare institution, e.g. hospital, especially subjects in intensive care or critical care (i.e. those units concerned with the provision of life support or organ support systems to patients); subjects on respiratory ventilators; subjects suffering from trauma; subjects with burns, subjects with acute and/or chronic wounds; neonatal subjects; elderly subjects; subjects with cancer (defined broadly herein to include any neoplastic condition; malignant or non-malignant), especially those with cancers of the immune system (e.g. leukaemias, lymphomas and other haematological cancers); subjects suffering from auto-immune conditions such as rheumatoid arthritis, diabetes mellitus type I, Crohn's disease, especially those undergoing immunosuppression treatment for those diseases; subjects with reduced or abrogated epithelial or endothelial secretion (e.g. mucous, tears, saliva) and/or secretion clearance (e.g. subjects with poorly functioning cilia on mucosal tissue and/or patients with hyperviscous mucous (e.g. smokers and subjects with COPD, COAD, COAP, bronchitis, cystic fibrosis, emphysema, lung cancer, asthma, pneumonia or sinusitis)) and subjects fitted with a medical device.

The bacitracin-alginate oligomer conjugates of the invention may be administered to the subject in any convenient form or by any convenient means in order to deliver effective amounts to the bacteria of the target infection and/or to the site carrying the invention or the site at risk of infection, e.g. by topical, enteral (e.g. oral, buccal, sublingual, rectal), parenteral (e.g. intravenous, intraspinal, intramuscular, subcutaneous), routes or by inhalation (including nasal inhalation). Administration may achieve systemic distribution or localised distribution, by which it is meant that delivery is effected to the bacteria of the target infection and/or to the site carrying the infection or to the site at risk of infection, but essentially no other location in the patient. The skilled person would be able to select an appropriate administration means to suit any particular target infection and/or site carrying the infection or site at risk of infection. Localised administration, particularly achieved by topical administration, may be preferred.

The skilled man will be able to formulate the bacitracin-alginate oligomer conjugates of the invention into pharmaceutical compositions that are adapted for these routes of administration and body distribution according to any of the conventional methods known in the art and widely described in the literature.

More specifically, the bacitracin-alginate oligomer conjugates of the invention may be incorporated, optionally together with other active agents, with one or more conventional carriers, diluents and/or excipients, to produce conventional galenic preparations such as tablets, pills, granules (e.g. in free form or enclosed in capsules), powders (e.g. inhalable powders, including dry inhalable powders), lozenges, sachets, cachets, elixirs, suspensions, emulsions, solutions, syrups, aerosols (as a solid or in a liquid medium), sprays (e.g. nasal sprays), compositions for use in nebulisers, ointments, creams, salves, soft and hard gelatine capsules, suppositories, pessaries, sterile injectable solutions, sterile packaged powders, and the like. Enteric coated solid or liquid compositions, e.g. enteric coated tablets and enteric coated granules (which may be provided in an enteric-coated capsule or in a non-enteric-coated capsule i.e. in which the coating may or may not be an enteric coating); sterile inhalable and sterile injectable compositions are of particular note.

Examples of suitable carriers, excipients, and diluents are lactose, dextrose, sucrose, sorbitol, mannitol, starches, gum acacia, calcium phosphate, inert alginate polymers, tragacanth, gelatine, calcium silicate, microcrystalline cellulose, polyvinylpyrrolidone, cellulose, water syrup, water, water/ethanol, water/ glycol, water/polyethylene, hypertonic salt water, glycol, propylene glycol, methyl cellulose, methylhydroxybenzoates, propyl hydroxybenzoates, talc, magnesium stearate, mineral oil or fatty substances such as hard fat or suitable mixtures thereof. Excipients and diluents of note are mannitol and hypertonic salt water (saline).

The compositions may additionally include lubricating agents, wetting agents, emulsifying agents, suspending agents, preserving agents, sweetening agents, flavouring agents, and the like.

For topical administration the bacitracin-alginate oligomer conjugates can be incorporated into creams, ointments, gels, salves, transdermal patches and the like. Further topical systems that are envisaged to be suitable are in situ drug delivery systems, for example gels where solid, semi-solid, amorphous or liquid crystalline gel matrices are formed in situ and which may comprise the alginate oligomer (which may be any alginate oligomer as herein defined). Such matrices can conveniently be designed to control the release of the bacitracin-alginate oligomer conjugates from the matrix, e.g. release can be delayed and/or sustained over a chosen period of time. Such systems may form gels only upon contact with biological tissues or fluids, e.g. mucosal surfaces. Typically the gels are bioadhesive and/or mucoadhesive. Delivery to any body site that can retain or be adapted to retain the pre-gel composition can be targeted by such a delivery technique. Such systems are described in WO 2005/023176, which is explicitly cited herein.

Parenterally administrable forms, e.g. solutions suitable for delivery intravenously, should be sterile and free from physiologically unacceptable agents, and should have low osmolarity to minimize irritation or other adverse effects upon administration and thus solutions should preferably be isotonic or slightly hypertonic, e.g. hypertonic salt water (saline). Suitable vehicles include aqueous vehicles customarily used for administering parenteral solutions such as sterile water for injection, Sodium Chloride Injection, Ringer's Injection, Dextrose Injection, Dextrose and Sodium Chloride Injection, Lactated Ringer's Injection and other solutions such as are described in Remington's Pharmaceutical Sciences, 15th ed., Easton: Mack Publishing Co., pp. 1405-1412 and 1461-1487 (1975) and The National Formulary XIV, 14th ed. Washington: American Pharmaceutical Association (1975)), which is explicitly cited herein.

The solutions can contain preservatives, antimicrobial agents, buffers and antioxidants conventionally used for parenteral solutions, excipients and other additives which are compatible with the bacitracin-alginate oligomer conjugates and which will not interfere with the manufacture, storage or use of products.

Simple sterile solutions of bacitracin-alginate oligomer conjugates or simple sterile liquid compositions comprising bacitracin-alginate oligomer conjugates may be especially convenient for use during surgical procedures and for delivery to the lungs, e.g. by nebuliser, or to the paranasal sinuses, e.g. by a nasal spray device.

Solid or liquid formulations of the bacitracin-alginate oligomer conjugates may be provided with an enteric coating that prevents degradation in the stomach and/or other parts of the upper GI tract but permits degradation in the lower GI tract, e.g. the small intestine. Such coatings are routinely prepared from polymers including fatty acids, waxes, shellac, plastics, and plant fibres. Specific examples thereof include but are not limited to methyl acrylate-methacrylic acid copolymers, methyl methacrylate-methacrylic acid copolymers, cellulose acetate succinate, hydroxypropyl methylcellulose phthalate, hydroxypropyl methylcellulose acetate succinate (hypromellose acetate succinate), polyvinyl acetate phthalate (PVAP), cellulose acetate trimellitate, and sodium alginate polymer. Enteric coated tablets and enteric coated granules (which may be provided in an enteric-coated capsule or in a non-enteric coated capsule) are of particular note. Enteric coated granules may be prepared in accordance with the teachings of WO 1989008448 and Al-Khedairy, E.B.H, 2006, Iraqi J.Pharm.Sci., Vol.15 (1) 49, the contents of which are cited herein, although the skilled person would be aware of further alternative techniques which may be used.

The relative content of the bacitracin-alginate oligomer conjugates in the compositions of the invention can vary depending on the dosage required and the dosage regime being followed but will be sufficient to achieve an effective amount at the target treatment site, i.e. the bacteria of the target infection and/or the site carrying the infection, or the site at risk of infection, taking account of variables such as the physical size of the subject to be treated, the nature of the subject's particular ailments, and the location and identity of the target treatment area. The skilled man would know that the amounts of the bacitracin-alginate oligomer conjugates can be reduced if a multiple dosing regime is followed or increased to minimise the number of administrations or applications.

A representative topical formulation, e.g. a cream, ointment or salve, which may be used to administer a bacitracin-alginate oligomer conjugate of the invention to the skin or cervix or other parts of the lower female reproductive system might contain 1 to 25%, 1 to 20%, 1 to 15%, 1 to 10%, 1 to 9%, 1 to 8%, 1 to 7%, 1 to 6%, 5 to 25%, 5 to 20%, 5 to 15%, 5 to 10%, 5 to 9%, 5 to 8%, 5 to 7%, 5 to 6%, 8 to 25%, 8 to 20%, 8 to 15%, 8 to 10%, 9 to 25%, 9 to 20%, or 9 to 15% w/v of the bacitracin-alginate oligomer conjugate, the remainder being comprised of pharmaceutically acceptable excipients, and/or other active agents if being used. Delivery devices designed for the application of topical formulations to the female reproductive system are known and may be employed to deliver the above mentioned formulations if convenient.

A pessary may be used to administer a bacitracin-alginate oligomer conjugate of the invention to the lower parts of the female reproductive tract. A representative formulation may contain 1 to 25%, 1 to 20%, e.g. 1 to 15%, 1 to 10%, 1 to 9%, 1 to 8%, 1 to 7%, 1 to 6%, 5 to 25%, 5 to 20%, 5 to 15%, 5 to 10%, 5 to 9%, 5 to 8%, 5 to 7%, 5 to 6%, 8 to 25%, 8 to 20%, 8 to 15%, 8 to 10%, 9 to 25%, 9 to 20%, or 9 to 15% w/v or w/w of the bacitracin-alginate oligomer conjugate, the remainder being comprised of pharmaceutically acceptable excipients, including solid excipients, and/or other active agents if being used. Rectal suppositories may be formulated similarly.

For administration to the nose or paranasal sinuses a sterile aqueous and/or oil-based liquid formulation (e.g. an emulsion) may be used; administered for instance by a nasal spray device, e.g. propellant-free or propellant-assisted. A representative formulation may contain 1 to 25%, 1 to 20%, e.g. 1 to 15%, 1 to 10%, 1 to 9%, 1 to 8%, 1 to 7% or 1 to 6%, 5 to 25%, 5 to 20%, 5 to 15%, 5 to 10%, 5 to 9%, 5 to 8%, 5 to 7%, 5 to 6%, 8 to 25%, 8 to 20%, 8 to 15%, 8 to 10%, 9 to 25%, 9 to 20%, or 9 to 15% w/v or w/w of the bacitracin-alginate oligomer conjugate, the remainder being comprised of pharmaceutically acceptable excipients, e.g. water, and/or other active agents if being used.

A representative inhalable solution to be used to administer a bacitracin-alginate oligomer conjugate of the invention to the upper respiratory tract typically will be sterile and may contain 6 to 25%, e.g. 6 to 20%, 6 to 15%, 6 to 10%, 8 to 25%, 8 to 20%, 8 to 15 %, 9 to 25%, 9 to 20%, 9 to 15 %, 10 to 15%, 10 to 20%, 10 to 25%, 15 to 20% or 15 to 25% w/v of the bacitracin-alginate oligomer conjugate, the remainder being comprised of pharmaceutically acceptable excipients, e.g. water, and/or other active agents if being used.

A representative inhalable powder to be used to administer a bacitracin-alginate oligomer conjugates of the invention to the lower respiratory tract may contain up to 90%, e.g. up to 85%, 80%, 75% or 70%, e.g. 50 to 90%, 55 to 90%, 60 to 90%, 65 to 90%, 70 to 90%, 75 to 90%, 80 to 90%, 85 to 90%, 50 to 85%, 55 to 85%, 60 to 85%, 65 to 85%, 70 to 85%, 75 to 85%, 80 to 85%, 50 to 80%, 55 to 80%, 60 to 80%, 65 to 80%, 70 to 80%, 75 to 80%, 50 to 70%, 55 to 70%, 60 to 70%, or 65 to 70% w/v or w/w of the bacitracin-alginate oligomer conjugate, the remainder being comprised of pharmaceutically acceptable excipients and/or other active agents if being used in the same composition.

In other embodiments a slow, delayed or sustained release formulations may be used for delivery, e.g. to the nose or paranasal sinuses. A representative formulation may be a powder containing the bacitracin-alginate oligomer conjugate or a suspension of said powder, said powder containing up to 90%, e.g. up to 85%, 80%, 75% or 70%, e.g. 50 to 90%, 55 to 90%, 60 to 90%, 65 to 90%, 70 to 90%, 75 to 90%, 80 to 90%, 85 to 90%, 50 to 85%, 55 to 85%, 60 to 85%, 65 to 85%, 70 to 85%, 75 to 85%, 80 to 85%, 50 to 80%, 55 to 80%, 60 to 80%, 65 to 80%, 70 to 80%, 75 to 80%, 50 to 70%, 55 to 70%, 60 to 70%, or 65 to 70% w/v or w/w of the bacitracin-alginate oligomer conjugate, the remainder being comprised of pharmaceutically acceptable excipients and/or other active agents if being used. The powder may comprise a coating that controls release of the bacitracin-alginate oligomer conjugate.

A representative tablet to be used to administer a bacitracin-alginate oligomer conjugate of the invention to the lower GI tract may contain up to 99%, up to 95%, 90%, 85% or 80%, e.g. 50 to 95%, 55 to 95%, 60 to 95%, 65 to 95%, 70 to 95%, 75 to 95%, 80 to 95%, 85 to 95%, 90 to 95%, 50 to 90%, 50 to 90%, 55 to 90%, 60 to 90%, 65 to 90%, 70 to 90%, 75 to 90%, 80 to 90%, 85 to 90%, 50 to 90%, 55 to 85%, 60 to 80% or, 65 to 75% w/v or w/w of the bacitracin-alginate oligomer conjugate, the remainder being comprised of pharmaceutically acceptable excipients and/or other active agents if being used. The tablet may be a multi-layered tablet.

An enteric coated tablet may also be effective in administering a bacitracin-alginate oligomer conjugate of the invention to the lower GI tract. A representative enteric coated tablet may contain up to 95%, e.g. up to 90%, 85% or 80%, e.g. 55 to 90%, 60 to 90%, 65 to 90%, 70 to 90%, 75 to 90%, 80 to 90%, 85 to 90%, 55 to 85%, 60 to 85%, 65 to 85%, 70 to 85%, 75 to 85%, 80 to 85%, 50 to 80%, 55 to 80%, 60 to 80%, 65 to 80%, 70 to 80%, or 75 to 80% w/v or w/w of the bacitracin-alginate oligomer conjugate, the remainder being comprised of pharmaceutically acceptable excipients, including the enteric coating (e.g. polymers including fatty acids, waxes, shellac, plastics, and plant fibres) and/or other active agents if being used. The tablet may be a multi-layered tablet, e.g. as described above.

Enteric coated granules may also be effective in administering a bacitracin-alginate oligomer conjugate of the invention to the lower GI tract. Such granules may be provided in a capsule which itself may or may not be provided with an enteric coating. A representative enteric coated granule may contain up to 95%, e.g. up to 90%, 85% or 80%, e.g. 55 to 90%, 60 to 90%, 65 to 90%, 70 to 90%, 75 to 90%, 80 to 90%, 85 to 90%, 55 to 85%, 60 to 85%, 65 to 85%, 70 to 85%, 75 to 85%, 80 to 85%, 50 to 80%, 55 to 80%, 60 to 80%, 65 to 80%, 70 to 80%, or 75 to 80% w/v or w/w of the bacitracin-alginate oligomer conjugate, the remainder being comprised of pharmaceutically acceptable excipients, including the enteric coating (e.g. polymers including fatty acids, waxes, shellac, plastics, and plant fibres) and/or other active agents if being used.

A representative aqueous solution for delivery of the bacitracin-alginate oligomer conjugate of the invention by injection (e.g. by intravenous, intraspinal, intramuscular or subcutaneous injection) will be sterile and may contain 6 to 25%, e.g. 6 to 20%, 6 to 15%, 6 to 10%, 8 to 25%, 8 to 20%, 8 to 15 %, 9 to 25%, 9 to 20%, 9 to 15 %, 10 to 15%, 10 to 20%, 10 to 25%, 15 to 20%, or 15 to 25% w/v of the bacitracin-alginate oligomer conjugate, the remainder being comprised of water and pharmaceutically acceptable excipients and/or other active agents if being used.

The bacitracin-alginate oligomer conjugates may be used at a daily dose of 0.1g to 10g, e.g. 0.5g to 5g, 0.8g to 3g, 1g to 2g, e.g. about 2g, which may be administered at one or more times per day (e.g. bis daily) and in one or more dosage forms or administration events (e.g. two tablets bis daily).

The bacitracin-alginate oligomer conjugates as defined herein may be used in conjunction or combination with one or more further therapeutically active agents, which may include other anti-microbial (e.g. antibacterial, antibiotic, antifungal and antiviral) agents, immunostimulatory agents, corticosteroids, non-steroidal antiinflammatory drugs (NSAIDs), bronchodilators, mucus viscosity-reducing agents (i.e. an agent which reduces the viscosity of mucus and which terms are used interchangeably with the term "mucolytic agent") or CFTR modulators (also known as "CFTR modifiers").

The further agent, e.g. antibiotic, may be unconjugated to an alginate oligomer or to any other conjugating moiety.

The bacitracin-alginate oligomer conjugate and the further therapeutically active agent may, for example, be administered together, in a single pharmaceutical formulation or composition, or separately (i.e. separate, sequential or simultaneous administration). Thus, bacitracin-alginate oligomer conjugate and the further therapeutically active agent may be combined, e.g. in a pharmaceutical kit or as a combined ("combination") product.

Thus a further aspect of the invention provides a product (e.g. a pharmaceutical combination or a kit) comprising a bacitracin-alginate oligomer conjugate as defined herein together with a further therapeutically active agent (e.g. those described above) as combined preparation for separate, sequential or simultaneous use in treating or preventing a bacterial infection, preferably a Gram negative bacterial infection, in a subject.

More generally this aspect of the invention also provides a kit comprising a bacitracin-alginate oligomer conjugate as defined herein together with a further therapeutically active agent (e.g. those described above).

Combinations comprising a bacitracin-alginate oligomer conjugate as herein defined and an antibiotic, an antifungal, a CFTR modulator and/or a mucus viscosity reducing agent are especially preferred. Such pharmaceutical products and pharmaceutical compositions are preferably adapted for use in the medical methods of the invention.

The further therapeutically active agent may conveniently be applied before, simultaneously with or following the bacitracin-alginate oligomer conjugate. Conveniently the further therapeutically active agent is applied at substantially the same time as the bacitracin-alginate oligomer conjugate or afterwards. In other embodiments the further therapeutically active agent may conveniently be applied or administered before the bacitracin-alginate oligomer conjugate. The further therapeutically active agent can also be given (e.g. administered or delivered) repeatedly at time points appropriate for the agent used. The skilled person is able to devise a suitable dosage regimen. In long term treatments the bacitracin-alginate oligomer conjugate can also be used repeatedly. The bacitracin-alginate oligomer conjugate can be applied as frequently as the further therapeutically active agent, or more or less frequently. The frequency required may depend on the site or location in or on the patient to which the bacitracin-alginate oligomer conjugate is administered and also the overall nature of the clinical condition displayed by the particular patient undergoing treatment.

The bacitracin-alginate oligomer conjugate and the further therapeutically active agent may therefore be formulated together or separately, that is in the same or in different formulations or pharmaceutical compositions, and may be provided for administration by the same or different routes. The use of bacitracin-alginate oligomer conjugate as herein defined to manufacture such pharmaceutical products and pharmaceutical compositions for use in the medical methods of the invention is also contemplated.

Representative antibiotics which may be used in conjunction or combination with the bacitracin-alginate oligomer conjugates as defined herein include, but are not limited to, the aminoglycosides (e.g. amikacin, gentamicin, kanamycin, neomycin, netilmicin, streptomycin, tobramycin); the β-lactams (e.g. the carbecephems (e.g. loracarbef); the 1st generation cephalosporins (e.g. cefadroxil, cefazolin, cephalexin); 2nd generation cephalosporins (e.g. cefaclor, cefamandole, cephalexin, cefoxitin, cefprozil, cefuroxime); 3rd generation cephalosporins (e.g. cefixime, cefdinir, cefditoren, cefoperazone, cefotaxime, cefpodoxime, ceftazidime, ceftibuten, ceftizoxime, ceftriaxone); 4th generation cephalosporins (e.g. cefepime); the monobactams (e.g. aztreonam); the macrolides (e.g. azithromycin, clarithromycin, dirithromycin, erythromycin, troleandomycin); the monobactams (e.g. aztreonam); the penicillins (e.g. amoxicillin, ampicillin, carbenicillin, cloxacillin, dicloxacillin, nafcillin, oxacillin, penicillin G, penicillin V, piperacillin, ticarcillin); the polypeptide antibiotics (e.g. colistin, polymyxin B, but in certain embodiments not bacitracin); the quinolones (e.g. ciprofloxacin, enoxacin, gatifloxacin, levofloxacin, lomefloxacin, moxifloxacin, norfloxacin, ofloxacin, trovafloxacin); the sulfonamides (e.g. mafenide, sulfacetamide, sulfamethizole, sulfasalazine, sulfisoxazole, trimethoprim- sulfamethoxazole); the tetracyclines (e.g. demeclocycline, doxycycline, minocycline, oxytetracycline, tetracycline); the glycylcyclines (e.g. tigecycline); the carbapenems (e.g. imipenem, meropenem, ertapenem, doripenem, panipenem/betamipron, biapenem, PZ-601); other antibiotics include chloramphenicol; clindamycin, ethambutol; fosfomycin; isoniazid; linezolid; metronidazole; nitrofurantoin; pyrazinamide; quinupristin/dalfopristin; rifampin; spectinomycin; and vancomycin.

Representative antifungals include, but are not limited to the polyenes (e.g. natamycin, rimocidin, filipin, nystatin, amphotericin B, candicin; the imidazoles (e.g. miconazole, ketoconazole, clotrimazole, econazole, bifonazole, butoconazole, fenticonazole, isoconazole, oxiconazole, sertaconazole, sulconazole, tioconazole); the triazoles (e.g. fluconazole, itraconazole, isavuconazole, ravuconazole, posaconazole, voriconazole,terconazole); the allylamines (e.g. terbinafine, amorolfine, naftifine, butenafine); and the echinocandins (e.g. anidulafungin, caspofungin, micafungin).

Representative antivirals include, but are not limited to abacavir, acyclovir, adefovir, amantadine, amprenavir, arbidol, atazanavir, atripla, boceprevir, cidofovir, combivir, darunavir, delavirdine, didanosine, docosanol, edoxudine, efavirenz, emtricitabine, enfuvirtide, entecavir, famciclovir, fomivirsen, fosamprenavir, foscarnet, fosfonet, ganciclovir, ibacitabine , imunovir, idoxuridine, imiquimod, indinavir, inosine, interferon type III, interferon type II, interferon type I, lamivudine, lopinavir, loviride, maraviroc, moroxydine, nelfinavir, nevirapine, nexavir, oseltamivir, penciclovir, peramivir, pleconaril, podophyllotoxin, raltegravir, ribavirin, rimantadine, ritonavir, saquinavir , stavudine, tenofovir, tenofovir disoproxil, tipranavir, trifluridine, trizivir, tromantadine, truvada, valaciclovir, valganciclovir, vicriviroc, vidarabine, viramidine, zalcitabine, zanamivir, and zidovudine.

Representative immunostimulatory agents include, but are not limited to, cytokines e.g. TNF, IL-1, IL-6, IL-8 and immunostimulatory alginates, such as high M -content alginates as described for example in US 5,169,840, WO91/11205 and WO03/045402 which are explicitly cited herein, but including any alginate with immunostimulatory properties.

Representative examples of suitable corticosteroids include but are not limited to prednisone, flunisolide, triamcinolone, fluticasone, budesonide, mometasone, beclomethasone, amcinonide, budesonide, desonide, fluocinonide, fluocinolone, halcinonide, hydrocortisone, cortisone, tixocortol, prednisolone, methylprednisolone, prednisone, betamethasone, dexamethasone, fluocortolone, aclometasone, prednicarbate, clobetasone, clobetasol, and fluprednidene.

Representative NSAIDs include, but are not limited to, the salicylates (e.g. aspirin (acetylsalicylic acid), choline magnesium trisalicylate, diflunisal, salsalate, the propionic acid derivatives (e.g. ibuprofen, dexibuprofen, dexketoprofen, fenoprofen, flurbiprofen, ketoprofen, loxoprofen, naproxen, oxaprozin), the acetic acid derivatives (e.g. aceclofenac, diclofenac, etodolac., indomethacin, ketorolac, nabumetone, tolmetin, sulindac), the enolic acid derivatives (e.g. droxicam, isoxicam, lornoxicam, meloxicam, piroxicam, tenoxicam), the anthranilic acid derivatives (e.g. flufenamic acid, meclofenamic acid, mefenamic acid, tolfenamic acid) and the selective COX-2 inhibitors (Coxibs; e.g. celecoxib, etoricoxib, lumiracoxib, parecoxib, rofecoxib, valdecoxib). The propionic acid derivatives (e.g. ibuprofen, dexibuprofen, dexketoprofen, fenoprofen, flurbiprofen, ketoprofen, loxoprofen, naproxen, oxaprozin) are preferred, ibuprofen being most preferred.

Representative examples of suitable bronchodilators include but are not limited to the β2 agonists (e.g. the short-acting β2 agonists (e.g. pirbuterol, epinephrine, salbutamol, levosalbutamol, clenbuterol, terbutaline, procaterol, metaproterenol, fenoterol, bitolterol mesylate, ritodrine, isoprenaline); the long-acting β2 agonists (e.g. salmeterol, formoterol, bambuterol, clenbuterol); and the ultra-long-acting β2 agonists (e.g. indacaterol)), the anticholinergics (e.g. ipratropium, oxitropium, tiotropium) and theophylline.

As used herein, the terms "mucolytic agent" and "mucus viscosity reducing agent" are intended to encompass agents which reduce the intrinsic viscosity of mucus and agents which reduce the attachment of mucus to underlying epithelium, in particular agents which directly or indirectly disrupt the molecular interactions within or between the components of mucus, agents which affect the hydration of mucus and agents which modulate the ionic microenvironment of the mucosal epithelium (particularly the levels of divalent cations, e.g. calcium). Representative examples of suitable mucus viscosity reducing agents include, but are not limited to, a nucleic acid cleaving enzyme (e.g. a DNase such as DNase I or dornase alfa), hypertonic saline, gelsolin, a thiol reducing agent, an acetylcysteine, an uncharged low molecular weight polysaccharide (e.g. dextran, mannitol), arginine (or other nitric oxide precursors or synthesis stimulators), an agonist of the P2Y2 subtype of purinergic receptors (e.g. denufosol) or an anionic polyamino acid (e.g. poly ASP or poly GLU). Ambroxol, bromhexine, carbocisteine, domiodol, eprazinone, erdosteine, letosteine, mesna, neltenexine, sobrerol, stepronin, tiopronin are specific mucolytics of note. DNase I and hypertonic saline are preferred.

CFTR modulators are small molecules which can redress, at least partially, a CFTR dysfunction. Present CFTR modulators fall into three main groups: CFTR potentiators, CFTR correctors and read-through agents (Derichs, N., Eur. Respir. Rev., 2013, 22(127), 58-65; Petit, R.S. and Fellner, C., Pharmacy and Therapeutics, 2014, 39(7), 500-511).

CFTR potentiators are CFTR modulators which increase the activity of the CFTR ion channel present on the epithelial cell surface. CFTR correctors are CFTR modulators which increase the amount of CFTR protein delivered or retained at the epithelial cell surface. Read-through agents (also known as "premature stop codon suppressors" (PSC suppressors) or "premature termination codon suppressors" (PTC suppressors, which terms are used interchangeably herein) are CFTR modulators which cause the translation machinery of the cell to pass over any premature termination codon in the CFTR mRNA thereby increasing the amount of substantially full length and functional CFTR produced.

Representative examples of suitable CFTR potentiators include, but are not limited to, ivacaftor (VX-770; N-(2,4-di-tert-butyl-5-hydroxyphenyl)-1,4-dihydro-4-oxoquinoline-3-carboxamide) and VRT-532 (4-methyl-2-(5-phenyl-1H-pyrazol-3-yl)-phenol) of Vertex Pharmaceuticals^{™}).

Representative examples of suitable CFTR correctors include, but are not limited to, Prototypical lumacaftor (VX-809) and VX-661 of Vertex Pharmaceuticals^{™} and N6022 (3-[1-(4-carbamoyl-2-methylphenyl)-5-(4-imidazol-1-ylphenyl)pyrrol-2-yl]propanoic acid).

Representative examples of suitable read-through agents include, but are not limited to, ataluren (PTC124) of PTC Therapeutics and gentamicin.

The invention will be further described with reference to the following Examples.

### EXAMPLES

### Example 1 - Preparation of bacitracin-alginate oligomer conjugates

### Preparation of alginate oligomers by acid hydrolysis

Sodium alginate, containing >60% guluronate monomers (PRONOVA UP MVG), was dissolved in dH₂O (10 mg/mL), the solution adjusted to 0.01 M HCI and then placed in a water bath at 80°C for up to 24 h. To terminate the hydrolysis, the acid hydrolysate was cooled and pH was increased to pH 7 by addition of 10 M NaOH. Solutions were lyophilised and re-suspended in minimal dH₂O.

To obtain acid-hydrolysed G-fragments (AHGs) with acceptable number average degree of polymerisation as well as molecular weight distribution, the solutions were filtered once by dialysis to remove LMW oligomers and salts. Typically, the following reaction times and dialysis membrane cut-offs were used to produce AHGs with a desired molecular weight:

| **Desired MW (g/mol)** | **Approx. reaction time (h:min)** | **Dialysis membrane cut-off (g/mol)** |
|---|---|---|
| 2,500 | 21:00 | 1,000 |
| 7,500 | 9:45 | 2,000 |
| 15,000 | 5:30 | 8,000 |

The final products were lyophilised and characterised by FT-IR and gel permeation chromatography (GPC). The GPC system comprised of two TSK G5000PW_{XL} and G3000PW_{XL} columns (Polymer Laboratories, UK) in series, mobile phase PBS (pH 7.4) and flow rate of 1 mL/min. Molecular weight and polydispersity were calculated relative to pullulan standards. Alginate standards are not commercially available, which necessitated the use of pullulan. These calibration standards do not give correct molecular weight for alginates since GPC separates according to hydrodynamic volume, which differs with varying macromolecular architecture. Alginates are more expanded than pullulans, resulting in a six-fold overestimate of alginate's molecular weight by GPC with pullulan. Consequently, a scaling factor (divide by 6) was applied to the apparent molecular weights calculated from pullulan calibration, to adjust for different macromolecular architecture of pullulan MW standards (Andersen T., et al., 2012, Alginates as biomaterials in tissue engineering. Carbohydrate Chemistry: Chemical and Biological Approaches, Vol. 37, 232-233*)*.

Samples for GPC were prepared in PBS (3 mg/mL) and the eluate was monitored using a differential refractometer (Optilab t-Rex (Wyatt, UK)). PL Caliber Instrument software, version 7.0.4, from Polymer Laboratories (UK) was used for data analysis.

Acid-hydrolysed G-fragments (AHGs) were generated with molecular weights between 2,500-26,000 g/mol (polydispersity 2.5-3.5). Typical characteristics of AHGs used in these studies are summarised in the table below.

| **Name** | **Apparent molecular weight (g/mol)*** | **PDI** | **DPₙ** |
|---|---|---|---|
| OligoG | 15,600 [2,600] | 1.8 | 13 |
| '2.5k' | 15,000 [2,500] | 1.4 | 13 |
| '6k' | 36,207 *[6,035]* | 4.0 | 30 |
| '7.5k' | 42,000 [7,000] | 2.6 | 35 |
| '9k' | 52,000 *[8,670]* | 3.0 | 45 |
| '13.5k' | 81,543 *[13,590]* | 2.9 | 68 |
| '15k' | 72,000 [12,000] | 3.5 | 60 |
| '16k' | 93,500 *[15,580]* | 3.6 | 81 |
| '20k' | 119,362 *[19,894]* | 3.1 | 100 |
| '26k' | 152,500 *[25,420]* | 3.5 | 132 |
| PRONOVA UP MVG⁺ | 651,000 *[108,500]* | 2.5 | 565 |

| | | | |
|---|---|---|---|
| *relative to pullulan MW standards; *value in [ ] indicates estimated actual MW after applying scaling factor to adjust for different macromolecular architecture of pullulan MW standards.* ⁺*Elutes in void volume, therefore MW is likely underestimated.* | | | |

OligoG CF-5/20 for conjugation was produced as described previously (Khan et al. 2012, Antimicrobial Agents and Chemotherapy 56(10), 5134-5141). OligoG is a 5-20mer alginate oligomer with at least 85%G residues.

### Ester conjugation ('E')

Briefly, for 2,500 g/mol AHG conjugation to bacitracin, alginate oligomer (200 mg, 0.08 mmol) was dissolved under stirring in anhydrous DMF (4 mL) (or anhydrous DMSO for Batches 3 and later) in a 10 mL round-bottomed flask. To this, DCC (16.5 mg, 0.08 mmol), DMAP (1.6 mg, 0.01 mmol) and bacitracin (37.5 mg, 0.03 mmol; Sigma) were added, and the reaction allowed to proceed at room temperature overnight, under stirring. To stop the reaction, the mixture was poured into excess chloroform (up to 20 mL), then the resulting precipitate was collected by filtration, re-dissolved in distilled water (dH₂O, 2 mL) and purified by FPLC (see below).

### Amide conjugation ('A')

Briefly, for 2,500 g/mol AHG conjugation to bacitracin, alginate oligomer (200 mg, 0.08 mmol) was dissolved under stirring in dH₂O (2 mL) in a 10 mL round-bottomed flask. To this, EDC (19.9 mg, 0.1 mmol) and sulfo-NHS (22.6 mg, 0.1 mmol) were added, and the mixture was left stirring for 15 min. Subsequently, bacitracin (37.5 mg, 0.03 mmol) was added, and the reaction mixture was left stirring for 2 h at room temperature, the stored at -20 °C until purification by FPLC (see below).

### Purification of conjugates by FPLC

Conjugates were purified from the reaction mixture by fast protein liquid chromatography (FPLC) (AKTA FPLC; Amersham Pharmacia Biotech, UK) using a pre-packed HiLoad Superdex 75 16/600 PG column with a UV detector and data analysis using Unicorn 4.0 software (Amersham Pharmacia Biotech, UK). Samples of the reaction mixture (2 mL) were injected into a 2 µL loop using PBS (pH 7.4) at 0.5 mL/min as a mobile phase. Fractions (5 mL) were collected, dialysed against de-ionised water (8 water changes) and assayed for protein content (BCA assay) before pooling fractions containing conjugate. The final conjugate was lyophilised and stored at - 20 °C.

### Example 2 - Characterisation of alginate oligomer-bacitracin conjugates

### Purity, molecular weight and drug content

Alginate oligomer-bacitracin conjugates were characterised by FPLC and GPC to assess purity and estimate molecular weight, and the total drug content of the conjugate was determined by the BCA assay using free drug as calibrant.

The FPLC system described above for purification was used again for final conjugate characterisation, with a pre-packed Superdex 75 10/300 GL column. Samples (200 µL) were dissolved in PBS (pH 7.4) and injected into a 100 mL loop at 0.5 mL/min. The GPC system described above as used again for final conjugate characterisation, and the same scaling factor was applied.

### Results

Using these alginate oligomers, a library of bacitracin-alginate oligomer conjugates has been prepared, with a typical reaction yield of -40%, with molecular weights of 19,500-83,250 g/mol (relative to pullulan molecular weight standards); containing 1-7% w/w drug loading (equivalent to 3-5 oligomers: 1 bacitracin molecule).

### Batch 1

| **Conjugate** | **Initial drug ratio (% w/w)** | **Drug loading (% w/w) (% yield)** | **Molar ratio (1 antibiotic: x AHG)** | **Molecular weight (g/mol)** | **PDI** | **Free drug (%)** |
|---|---|---|---|---|---|---|
| OligoG-ESTER-bacitracin | 18.2 | 6.8 (37%) | 7.80 | 19,500 | 2.0 | 62.1 |
| 6k AHG-ESTER-bacitracin | 7.9 | 3.7 (47%) | 6.17 | 42,250 | 2.9 | 41.3 |
| 13.5k AHG-ESTER-bacitracin | 3.5 | 1.0 (29%) | 10.44 | 83,250 | 3.6 | 3.5 |

| | | | | | | |
|---|---|---|---|---|---|---|
| *Purified by ion exchange instead of size exclusion chromatography | | | | | | |

### Batch 2

| **Conjugate** | **Drug loading (% w/w)** |
|---|---|
| 7.5k AHG-A-bacitracin | 1.5 |
| 7.5k AHG-E-bacitracin | 0.7 |
| 15k AHG-A-bacitracin | 1.7 |

### Batch 4

| **Conjugate** | **Mn* (g/mol)** | **Mw* (g/mol)** | **PDI** | **Free drug (%)** | **Drug loading (% w/w)** |
|---|---|---|---|---|---|
| Bacitracin | 9,250 | 10,750 | 1.1 | | |
| OligoG-A-bacitracin | 10,750 | 27,250 | 2.5 | 0.7 | 4.3 |
| OligoG | 7,000 | 16,750 | 2.4 | | |

### Example 3 - Antimicrobial activity

### Bacterial isolates and strains:

The strains used for susceptibility testing include both culture collection strains and clinical isolates are recited below. Their known relevant genotypes and origin have been described by Khan et al. *supra.*

| **Code** | **Isolate** | **Description** |
|---|---|---|
| V2 | *Pseudomonas aeruginosa* (301) | MDR-PSA isolate, multi drug resistant isolate defined as being resistant to piperacillin, ceftazidime, imipenem, and gentamicin. Origin; Poland. Khan *et al., supra.* |
| V3 | *Klebsiella pneumoniae* | Khan *et al., supra.* |
| V5 | *Escherichia coli* | Khan *et al.,* supra. |
| V19 | *Acinetobacter baumannii* | Khan *et al., supra.* |
| E68 | *Staphylococcus aureus* NCTC 6571 | *Staphylococcus aureus* control Khan *et al., supra.* |
| E75 | *Staphylococcus aureus* NCTC 12493 | Methicillin-resistant *Staphylococcus aureus* (MRSA) control NCTC strain |

### Determination of antimicrobial activity

The MICs of alginate oligomers, bacitracin and alginate oligomer conjugates thereof were determined for isolates V2, V3, V5, V19, E68 and E75 using the broth microdilution method in accordance with standard guidelines (CLSI 2012). Test organisms were suspended in Mueller Hinton cation adjusted (MH) broth (100 µL, 1-5 x 10⁴ CFU/mL) and incubated in 96-well microtitre plates in serial two-fold dilutions of the test compounds. The MIC was defined as the lowest concentration of test compound that produced no visible growth after 16-20 hours. Unless otherwise stated, results show median values of 3 experiments.

### Results

None of the alginate fractions, or OligoG showed any antimicrobial activity, whereas unconjugated bacitracin showed high antimicrobial activity against *Staphylococcus aureus* (Gram positive) but not the Gram negative species tested (*Pseudomonas aeruginosa, Klebsiella pneumonia, Escherichia coli and Acinetobacter baumannii.*

On the hand alginate oligomer conjugated bacitracin shows good antimicrobial activity against (lower MIC values) against both the Gram negative strains (in particular the *E. coli* strain) and the Gram positive strains.

Antimicrobial activity was greatest for conjugates containing low molecular weight alginate oligomers (including OligoG).

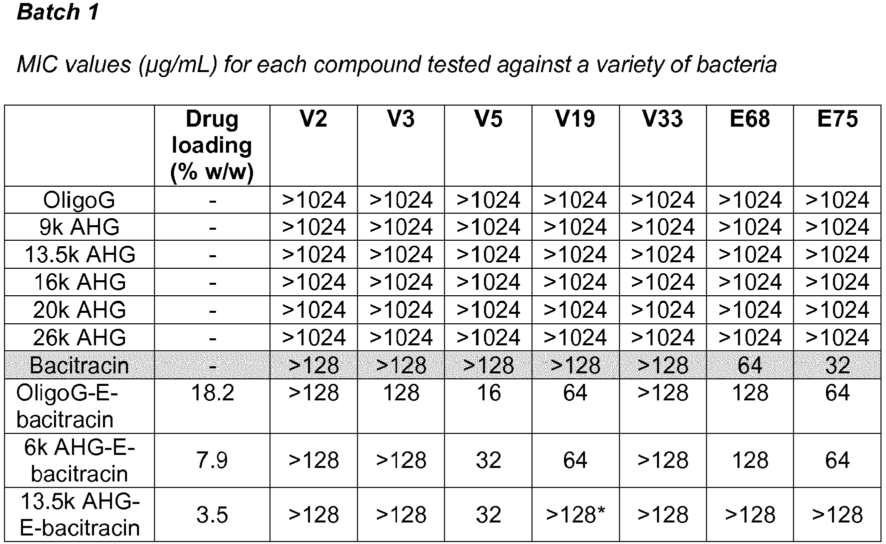

| | | | | | | | |
|---|---|---|---|---|---|---|---|
| ***Batch 2*** | | | | | | | |

| *MIC values (µg*/*mL) for each compound tested against a variety of bacteria (n*=*3, median)* | | | | | | | |
|---|---|---|---|---|---|---|---|
| **Conjugate** | **Drug loading (% w/w)** | **V2** | **V3** | **V5** | **V19** | **E68** | **E75** |
| Bacitracin | - | >8192 | >8192 | 640 | 640 | 40 | 20 |
| OligoG-A-bacitracin 3:1 | 3.2 | >256 | >256 | 32 | 128 | >256 | >256 |
| OligoG-E-bacitracin 3:1 | 0.9 | >256 | >256 | 16 | 128 | >256 | 128 |
| OligoG-A-bacitracin 2:1 | 1.1 | >256 | >256 | 32 | 256 | >256 | 128 |
| OligoG-E-bacitracin 2:1 | 1.9 | >256 | >256 | 16 | 128 | >256 | 256 |
| OligoG-A-bacitracin 1:1 | 5 | >256 | >256 | 64 | 256 | >256 | 256 |
| OligoG-E-bacitracin 1:1 | 0.6 | >256 | >256 | 64 | 256 | >256 | 128 |
| 7.5k AHG-A-bacitracin | 1.5 | >256 | >256 | 32 | 256 | >256 | >256 |
| 7.5k AHG-E-bacitracin | 0.7 | >128 | >128 | 128 | >128 | - | >256 |
| 15k AHG-A-bacitracin | 1.7 | >256 | >256 | 128 | 256 | 256 | >256 |
| OligoG-A-bacitracin 3:1 | 3.2 | >256 | >256 | 32 | 128 | >256 | >256 |
| OligoG-E-bacitracin 3:1 | 0.9 | >256 | >256 | 16 | 128 | >256 | 128 |

## Claims

1. A bacitracin-alginate oligomer conjugate comprising a bacitracin-class antibiotic connected covalently to at least one alginate oligomer via a direct covalent bond or a covalent molecular linker, wherein said alginate oligomer has 2 to 50 monomer residues, or a pharmaceutically acceptable salt, solvate, hydrate, diastereoisomer, tautomer or enantiomer thereof.

2. The bacitracin-alginate oligomer conjugate of claim 1, wherein said bacitracin-class antibiotic is
(i) selected from bacitracin A1, A2, B1, B2, B3, C, D1, D2, D3 and E and functionally equivalent derivatives thereof;
(ii) represented by Formula II 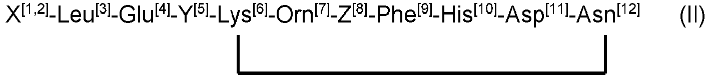
wherein Leu is leucine; Glu is glutamic acid; Lys is lysine; Orn is ornithine; Phe is phenylalanine; His is histidine; Asp is aspartic acid; Asn is asparagine;
Y is valine, isoleucine, leucine or 5-methylene-isoleucine;
Z is valine, isoleucine, leucine or 5-methylene-isoleucine; and
X is W^{[1]}-Cys^{[2]} or V^{[1]}-Thz^{[2]}; wherein
W is valine, isoleucine, leucine or 5-methylene-isoleucine and Cys is cysteine; and
V is
H₂N-C(R)H-
wherein R is the α side chain of valine, isoleucine, leucine or 5-methylene-isoleucine; and
Thz is a thiazoline ring 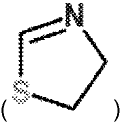 which is 2' coupled to V and 4' coupled to the α-carbon of Leu^{[3]}, wherein none or one or more, of amino acids Leu^{[3]}, Glu^{[4]}, Orn^{[7]}, Phe^{[9]}, His^{[10]} or Asp^{[11}is replaced by another amino acid residue which may be selected from natural or non-genetically encoded amino acids, preferably wherein said natural or non-genetically encoded amino acid is selected from leucine, threonine, acid, phenylalanine, arginine, histidine, lysine, asparagine, serine, cysteine, homolysine, ornithine, diaminobutyric acid (e.g. α,γ-diaminobutyric acid), diaminopimelic acid, diaminopropionic acid, homoarginine, trimethylysine, trimethylornithine, 4-aminopiperidine-4-carboxylic acid, 4-amino-1-carbamimidoylpiperidine-4-carboxylic acid and 4-guanidinophenylalanine;
(iii) selected from bacitracin A (A1 and/or A2) and bacitracin B (B1 and/or B2); and/or
(iv) complexed with divalent metal cations, e.g. Zn²⁺, Mg²⁺, Mn²⁺, and Co²⁺

3. The bacitracin-alginate oligomer conjugate of claim 1 or claim 2, wherein the alginate oligomer has a degree of polymerisation (DP), or a number average degree of polymerisation (DPn) of
(i) 4 to 50, 4 to 35, 4 to 30, 4 to 25, 4 to 22, 4 to 20, 4 to 18, 4 to 16 or 4 to 14,
(ii) 6 to 50, 6 to 35, 6 to 30, 6 to 25, 6 to 22, 6 to 20, 6 to 18, 6 to 16 or 6 to 14,
(iii) 8 to 50, 8 to 35, 8 to 30, 8 to 25, 10 to 25, 10 to 22, 10 to 20, 10 to 18, or 10 to 15, or
(iv) 20 to 50, 20 to 45, 20 to 40, 20 to 35, 20 to 30 or 20 to 25, or
(v) 30 to 50, 30 to 45, 30 to 40 or 30 to 35.

4. The bacitracin-alginate oligomer conjugate of any one of claims 1 to 3, wherein the alginate oligomer has at least 70% G residues, or at least 80%, or at least 85%, or at least 90%, or at least 95% G residues, preferably wherein at least 80% of the G residues are arranged in G-blocks.

5. The bacitracin-alginate oligomer conjugate of any one of claims 1 to 3, wherein the alginate oligomer has at least 70% M residues, or at least 80%, or at least 85%, or at least 90%, or at least 95% M residues, preferably wherein at least 80% of the M residues are arranged in M-blocks.

6. The bacitracin-alginate oligomer conjugate of any one of claims 1 to 5, wherein said direct covalent bond is part of an ester, carbonate ester, orthoester, ketal, hemiketal, ether, acetal, hemiacteal, peroxy, methylenedioxy, amide, amine, imine, imide, azide, azo, oxime, sulfide, disulfide, sulfinyl, sulfonyl, carbonothioyl, thioester, phosphine or phosphodiester functional group, preferably part of an ester or an amide.

7. The bacitracin-alginate oligomer conjugate of any one of claims 1 to 5, wherein said covalent linker is or comprises molecular groups selected from:
(i) an amino acid or a peptide;
(ii) a monosaccharide or an oligosaccharide other than guluronate or mannuronate or polymers formed therefrom;
(iii) a ribonucleotide or a deoxyribonucleotide;
(iv) a straight chain, branched or cyclic, substituted or unsubstituted, alkyl, alkenyl or alkynl group;
(v) an acetyl, succinyl, aconityl (*cis* or *trans*), glutaryl, methylsuccinyl, trimellityl cysteamine, penicillamine, N-(2-mercaptopropionyl)glycine, 2-mercaptopropionic acid, homocysteine, 3-mercaptopropionic acid or deamino-penicillamine group.

8. The bacitracin-alginate oligomer conjugate of any one of claims 1 to 7, wherein said direct covalent bond, a functional group containing said covalent bond or said covalent molecular linker is
(i) acid labile;
(ii) sensitive to reactive oxygen species; and/or
(iii) degraded by an enzyme secreted by a bacterium or an immune cell.

9. The bacitracin-alginate oligomer conjugate of any one of claims 1 to 8, wherein said conjugate consists of
(i) at least one alginate oligomer covalently bonded to a bacitracin-class antibiotic via an ester bond formed from a carboxyl group on the alginate and hydroxyl group on the bacitracin, or
(ii) at least one alginate oligomer covalently bonded to a bacitracin-class antibiotic via an amide bond formed from a carboxyl group on the alginate and an amine group on the bacitracin.

10. A pharmaceutical composition comprising a bacitracin-alginate oligomer conjugate as defined in any one of claims 1 to 9 and a pharmaceutically acceptable excipient, carrier or diluent.

11. A method for the preparation of a bacitracin-alginate oligomer as defined in any one of claims 1 to 9, said method comprising
(ia) providing an alginate oligomer, wherein said alginate oligomer has 2 to 50 monomer residues, and a bacitracin-class antibiotic and forming a direct covalent bond between two molecular groups thereon; or
(ib) providing an alginate oligomer, a bacitracin-class antibiotic and a covalent molecular linker and forming a direct covalent bond between two molecular groups on the alginate oligomer and the linker molecule and forming a direct covalent bond between two molecular groups on the bacitracin-class antibiotic and the linker molecule; or
(ic) providing an alginate oligomer and a bacitracin-class antibiotic wherein one or both carry a covalent molecular linker molecule covalently bonded thereto and covalently linking the alginate oligomer to the bacitracin-class antibiotic via at least one of the linker molecules; and optionally
(ii) separating at least a portion of the bacitracin-alginate oligomer conjugate from the reaction mixture.

12. The method of claim 11, said method comprising
(i) providing an aqueous solution of an alginate oligomer having an available carboxyl group wherein said alginate oligomer has 2 to 50 monomer residues
(ii) contacting said alginate solution with 1-ethyl-3-[3-dimethylaminopropyl]carbodiimide hydrochloride (EDC) in an amount and under conditions sufficient to activate at least one carboxyl group in the alginate oligomer;
(iii) optionally contacting said carboxyl activated alginate oligomer with sulfo N-hydroxysuccinimide (sulfo-NHS) in an amount and under conditions sufficient to form an amine-reactive sulfo-NHS ester;
(iv) contacting said carboxyl activated alginate oligomer of step (ii) or the amine-reactive sulfo-NHS ester of step (iii) with an bacitracin-class antibiotic having an available primary amine group in an amount and under conditions sufficient to form an amide bond between the alginate oligomer and the bacitracin-class antibiotic; and
(v) separating at least a portion of the bacitracin-alginate oligomer conjugate from the reaction mixture.

13. The method of claim 11, said method comprising
(i) providing a solution of an alginate oligomer having an available carboxyl group, wherein said alginate oligomer has 2 to 50 monomer residues, preferably an organic (e.g. DMF and/or DMSO) solution;
(ii) contacting said alginate solution with dicyclohexylcarbodiimide (DCC) in an amount and under conditions sufficient to form an O-acylisourea intermediate;
(iii) contacting said O-acylisourea intermediate with an bacitracin-class antibiotic having an available hydroxyl group and 4-N,N-dimethylaminopyridine (DMAP) in amounts and under conditions sufficient to form an ester bond between the alginate oligomer and the bacitracin-class antibiotic; and
(iv) separating at least a portion of the bacitracin-alginate oligomer conjugate from the reaction mixture;
wherein steps (ii) and (iii) may be performed simultaneously.

14. A bacitracin-alginate oligomer conjugate as defined in any one of claims 1 to 9 or a pharmaceutical composition as defined in claim 10 for use in the treatment of prevention of a bacterial infection, preferably wherein the bacterial infection is
(i) in a wound, preferably a chronic wound;
(ii) a respiratory infection in a subject suffering from an underlying respiratory disorder or condition, preferably selected from CF, COPD/COAD, or asthma;
(iii) a device related infection associated with implantable or prosthetic medical devices; or
(iv) a systemic infection or an infection of multiple loci within or on the subject.

15. The bacitracin-alginate oligomer conjugate or pharmaceutical composition for the use of claim 14, wherein the infection is a Gram negative bacterial infection.

## Patentansprüche

1. Bacitracin-Alginat-Oligomer-Konjugat, umfassend ein Antibiotikum der Bacitracin-Klasse, das über eine direkte kovalente Bindung oder einen kovalenten molekularen Linker kovalent an mindestens ein Alginat-Oligomer gebunden ist, wobei das Alginat-Oligomer 2 bis 50 Monomerreste aufweist, oder ein pharmazeutisch verträgliches Salz, Solvat, Hydrat, Diastereoisomer, Tautomer oder Enantiomer davon.

2. Bacitracin-Alginat-Oligomer-Konjugat nach Anspruch 1, wobei das Antibiotikum der Bacitracin-Klasse
(i) ausgewählt ist aus Bacitracin A1, A2, B1, B2, B3, C, D1, D2, D3 und E und funktionell äquivalenten Derivaten davon;
(ii) durch die Formel II dargestellt ist 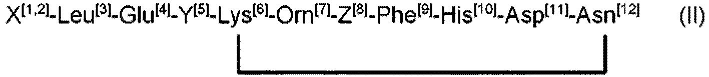
wobei Leu für Leucin steht; Glu für Glutaminsäure steht; Lys für Lysin steht; Orn für Ornithin steht; Phe für Phenylalanin steht; His für Histidin steht; Asp für Asparaginsäure steht; Asn für Asparagin steht;
Y für Valin, Isoleucin, Leucin oder 5-Methylen-Isoleucin steht;
Z für Valin, Isoleucin, Leucin oder 5-Methylen-Isoleucin steht; und
X für W^{[1]}-Cys^{[2]} oder V^{[1]}-Thz^{[2]}ist; steht
W für Valin, Isoleucin, Leucin oder 5-Methylen-Isoleucin steht und Cys für Cystein steht; und
V steht für
H₂N-C(R)H-
wobei R die α-Seitenkette von Valin, Isoleucin, Leucin oder 5-Methylen-Isoleucin ist; und
Thz ein Thiazolinring 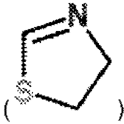 ist, der 2' an V und 4' gekoppelt ist an den α-Kohlenstoff von Leu ^{[3]}gekoppelt ist,
wobei keine oder eine oder mehrere der Aminosäuren Leu^{[3]}, Glu^{[4]}, Orn^{[7]}, Phe^{[9]}, His^{[10]} oder Asp^{[11} durch einen anderen Aminosäurerest ersetzt ist, der aus natürlichen oder nicht genetisch kodierten Aminosäuren ausgewählt werden kann, wobei die natürliche oder nicht genetisch kodierte Aminosäure bevorzugt ausgewählt ist aus Leucin, Threonin, Acid, Phenylalanin, Arginin, Histidin, Lysin, Asparagin, Serin, Cystein, Homolysin, Ornithin, Diaminobuttersäure (z. B. α,γ-Diaminobuttersäure), Diaminopimelinsäure, Diaminopropionsäure, Homoarginin, Trimethylysin, Trimethylornithin, 4-Aminopiperidin-4-carbonsäure, 4-Amino-1- carbamimidoylpiperidin-4-carbonsäure und 4-Guanidinophenylalanin;
(iii) ausgewählt ist aus Bacitracin A (A1 und/oder A2) und Bacitracin B(B1 und/oder B2); und/oder
(iv) komplexiert ist mit zweiwertigen Metallkationen, z. B. Zn²⁺, Mg²⁺, Mn²⁺, und Co²⁺

3. Bacitracin-Alginat-Oligomer-Konjugat nach Anspruch 1 oder Anspruch 2, wobei das Alginat-Oligomer einen Polymerisationsgrad (DP) oder einen zahlenmittleren Polymerisationsgrad (DPn) von
(i) 4 bis 50, 4 bis 35, 4 bis 30, 4 bis 25, 4 bis 22, 4 bis 20, 4 bis 18, 4 bis 16 oder 4 bis 14,
(ii) 6 bis 50, 6 bis 35, 6 bis 30, 6 bis 25, 6 bis 22, 6 bis 20, 6 bis 18, 6 bis 16 oder 6 bis 14,
(iii) 8 bis 50, 8 bis 35, 8 bis 30, 8 bis 25, 10 bis 25, 10 bis 22, 10 bis 20, 10 bis 18 oder 10 bis 15, oder
(iv) 20 bis 50, 20 bis 45, 20 bis 40, 20 bis 35, 20 bis 30 oder 20 bis 25, oder
(v) 30 bis 50, 30 bis 45, 30 bis 40 oder 30 bis 35 aufweist.

4. Bacitracin-Alginat-Oligomer-Konjugat nach einem der Ansprüche 1 bis 3, wobei das Alginat-Oligomer mindestens 70 % G-Reste oder mindestens 80 % oder mindestens 85 % oder mindestens 90 % oder mindestens 95 % G-Reste aufweist, bevorzugt wobei mindestens 80 % der G-Reste in G-Blöcken angeordnet sind.

5. Bacitracin-Alginat-Oligomer-Konjugat nach einem der Ansprüche 1 bis 3, wobei das Alginat-Oligomer mindestens 70 % M-Reste oder mindestens 80 % oder mindestens 85 % oder mindestens 90 % oder mindestens 95 % M-Reste aufweist, bevorzugt wobei mindestens 80 % der M-Reste in M-Blöcken angeordnet sind.

6. Bacitracin-Alginat-Oligomer-Konjugat nach einem der Ansprüche 1 bis 5, wobei die direkte kovalente Bindung Teil eines Esters, Carbonatesters, Orthoesters, Ketals, Hemiketals, Ethers, Acetals, Hemiacetals, Peroxys, Methylendioxy-, Amid-, Amin-, Imin-, Imid-, Azid-, Azo-, Oxim-, Sulfid-, Disulfid-, Sulfinyl-, Sulfonyl-, Carbonothioyl-, Thioester-, Phosphin- oder Phosphodiester-Funktionsgruppe ist, bevorzugt Teil eines Esters oder eines Amids.

7. Bacitracin-Alginat-Oligomer-Konjugat nach einem der Ansprüche 1 bis 5, wobei der kovalente Linker molekulare Gruppen ist oder umfasst, ausgewählt aus:
(i) einer Aminosäure oder einem Peptid;
(ii) einem Monosaccharid oder einem Oligosaccharid, das kein Guluronat oder Mannuronat ist, oder aus daraus gebildeten Polymeren;
(iii) einem Ribonukleotid oder einem Desoxyribonukleotid;
(iv) einer geradkettigen, verzweigten oder cyclischen, substituierten oder unsubstituierten Alkyl-, Alkenyl- oder Alkinylgruppe;
(v) einer Acetyl-, Succinyl-, Aconityl- (*cis oder trans*)*,* Glutaryl-, Methylsuccinyl-, Trimellityl-Cysteamin-, Penicillamin-, N-(2-Mercaptopropionyl)-Glycin-, 2-Mercaptopropionsäure-, Homocystein-, 3-Mercaptopropionsäure- oder Deamino-Penicillamin-Gruppe.

8. Bacitracin-Alginat-Oligomer-Konjugat nach einem der Ansprüche 1 bis 7, wobei die direkte kovalente Bindung, eine funktionelle Gruppe, die die kovalente Bindung enthält, oder der kovalente molekulare Linker
(i) säurelabil ist;
(ii) empfindlich gegenüber reaktiven Sauerstoffspezies ist; und/oder
(iii) durch ein von einem Bakterium oder einer Immunzelle sezerniertes Enzym abgebaut wird.

9. Bacitracin-Alginat-Oligomer-Konjugat nach einem der Ansprüche 1 bis 8, wobei das Konjugat besteht aus
(i) mindestens einem Alginat-Oligomer, das kovalent an ein Antibiotikum der Bacitracin-Klasse über eine Esterbindung gebunden ist, die aus einer Carboxylgruppe am Alginat und einer Hydroxylgruppe am Bacitracin gebildet wird, oder
(ii) mindestens einem Alginat-Oligomer, das kovalent an ein Antibiotikum der Bacitracin-Klasse über eine Amidbindung gebunden ist, die aus einer Carboxylgruppe am Alginat und einer Amingruppe am Bacitracin gebildet ist.

10. Pharmazeutische Zusammensetzung, umfassend ein Bacitracin-Alginat-Oligomer-Konjugat, wie in einem der Ansprüche 1 bis 9 definiert, und einen pharmazeutisch verträglichen Hilfsstoff, Träger oder Verdünnungsmittel.

11. Verfahren zur Herstellung eines Bacitracin-Alginat-Oligomers, wie in einem der Ansprüche 1 bis 9 definiert, wobei das Verfahren umfasst
(ia) Bereitstellen eines Alginat-Oligomers, wobei das Alginat-Oligomer 2 bis 50 Monomerreste aufweist, und eines Antibiotikums der Bacitracin-Klasse und Bilden einer direkten kovalenten Bindung zwischen zwei Molekülgruppen daran; oder
(ib) Bereitstellen eines Alginat-Oligomers, eines Antibiotikums der Bacitracin-Klasse und eines kovalenten molekularen Linkers und Bilden einer direkten kovalenten Bindung zwischen zwei molekularen Gruppen auf dem Alginat-Oligomer und dem Linker-Molekül und Bilden einer direkten kovalenten Bindung zwischen zwei molekularen Gruppen auf dem Antibiotikum der Bacitracin-Klasse und dem Linker-Molekül; oder
(ic) Bereitstellen eines Alginat-Oligomers und eines Antibiotikums der Bacitracin-Klasse, wobei eines oder beide ein kovalentes molekulares Linker-Molekül tragen, das kovalent daran gebunden ist, und kovalentes Binden des Alginat-Oligomers an das Antibiotikum der Bacitracin-Klasse über mindestens eines der Linker-Moleküle; und optional
(ii) Abscheiden mindestens eines Abschnitts des Bacitracin-Alginat-Oligomer-Konjugats aus dem Reaktionsgemisch.

12. Verfahren nach Anspruch 11, wobei das Verfahren umfasst
(i) Bereitstellen einer wässrigen Lösung eines Alginatoligomers, das eine verfügbare Carboxylgruppe aufweist, wobei das Alginatoligomer 2 bis 50 Monomerreste aufweist
(ii) Inkontaktbringen der Alginatlösung mit 1-Ethyl-3-[3-dimethylaminopropyl]carbodiimid-Hydrochlorid (EDC) in einer Menge und unter Bedingungen, die ausreichen, um mindestens eine Carboxylgruppe in dem Alginat-Oligomer zu aktivieren;
(iii) optional Inkontaktbringen des carboxylaktivierten Alginatoligomers mit Sulfo-N-Hydroxysuccinimid (Sulfo-NHS) in einer Menge und unter Bedingungen, die ausreichen, um einen aminreaktiven Sulfo-NHS-Ester zu bilden;
(iv) Inkontaktbringen des carboxylaktivierten Alginatoligomers aus Schritt (ii) oder des aminreaktiven Sulfo-NHS-Esters aus Schritt (iii) mit einem Antibiotikum der Bacitracin-Klasse, das eine verfügbare primäre Amingruppe in einer Menge und unter Bedingungen aufweist, die ausreichen, um eine Amidbindung zwischen dem Alginatoligomer und dem Antibiotikum der Bacitracin-Klasse zu bilden; und
(v) Abscheiden mindestens eines Abschnitts des Bacitracin-Alginat-Oligomer-Konjugats aus dem Reaktionsgemisch.

13. Verfahren nach Anspruch 11, wobei das Verfahren umfasst
(i) Bereitstellen einer Lösung eines Alginatoligomers, das eine verfügbare Carboxylgruppe aufweist, wobei das Alginatoligomer 2 bis 50 Monomerreste aufweist, bevorzugt eine organische Lösung (z.B. DMF und/oder DMSO);
(ii) Inkontaktbringen der Alginatlösung mit Dicyclohexylcarbodiimid (DCC) in einer Menge und unter Bedingungen, die zur Bildung eines O-Acylisoharnstoff-Zwischenprodukts ausreichen;
(iii) Inkontaktbringen des O-Acylharnstoff-Zwischenprodukts mit einem Antibiotikum der Bacitracin-Klasse, das eine verfügbare Hydroxylgruppe und 4-N,N-Dimethylaminopyridin (DMAP) in Mengen und unter Bedingungen aufweist, die ausreichen, um eine Esterbindung zwischen dem Alginat-Oligomer und dem Antibiotikum der Bacitracin-Klasse zu bilden; und
(iv) Abscheiden mindestens eines Abschnitts des Bacitracin-Alginat-Oligomer-Konjugats aus dem Reaktionsgemisch;
wobei die Schritte (ii) und (iii) gleichzeitig durchgeführt werden können.

14. Bacitracin-Alginat-Oligomer-Konjugat, wie in einem der Ansprüche 1 bis 9 definiert, oder eine pharmazeutische Zusammensetzung, wie in Anspruch 10 definiert, zur Verwendung bei der Behandlung oder Vorbeugung einer bakteriellen Infektion, bevorzugt wobei die bakterielle Infektion
(i) in einer Wunde, bevorzugt einer chronischen Wunde, vorliegt;
(ii) eine Atemwegsinfektion in einem Probanden ist, der an einer zugrundeliegenden Atemwegserkrankung oder einem zugrundeliegenden Atemwegszustand leidet, bevorzugt ausgewählt aus CF, COPD/COAD oder Asthma;
(iii) eine vorrichtungsbezogene Infektion ist, die implantierbaren oder prothetischen medizinischen Vorrichtungen zugeordnet ist; oder
(iv) eine systemische Infektion oder eine Infektion mehrerer Loci innerhalb oder auf dem Subjekt ist.

15. Bacitracin-Alginat-Oligomer-Konjugat oder pharmazeutische Zusammensetzung für die Verwendung nach Anspruch 14, wobei die Infektion eine Gram-negative bakterielle Infektion ist.

## Revendications

1. Conjugué oligomère d'alginate-bacitracine comprenant un antibiotique de classe bacitracine lié de manière covalente à au moins un oligomère d'alginate par l'intermédiaire d'une liaison covalente directe ou d'un lieur moléculaire covalent, dans lequel ledit oligomère d'alginate présente 2 à 50 résidus monomères, ou un sel, un solvant, un hydrate, un diastéréoisomère, un tautomère, ou un énantiomère pharmaceutiquement acceptable de celui-ci.

2. Conjugué oligomère d'alginate-bacitracine selon la revendication 1, dans lequel ledit antibiotique de classe bacitracine est
(i) sélectionné parmi la bacitracine A1, A2, B1, B2, B3, C, D1, D2, D3 et E et des dérivés fonctionnellement équivalents de celle-ci ;
(ii) représenté par la formule II 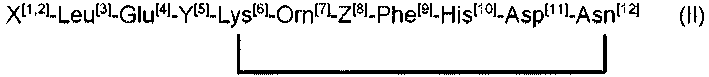
dans lequel Leu est leucine ; Glu est l'acide glutamique ; Lys est lysine; Orn est ornithine ; Phe est phénylalanine ; His est histidine ; Asp est l'acide aspartique ; Asn est asparagine ;
Y est valine, isoleucine, leucine ou 5-méthylène-isoleucine ;
Z est valine, isoleucine, leucine ou 5-méthylène-isoleucine ; et
X est W^{[1]}-Cys^{[2]} ou V^{[1]}-Thz^{[2]} ; dans lequel
W est valine, isoleucine, leucine ou 5-méthylène-isoleucine et Cys est cystéine ; et V est
H₂N-C(R)H-
dans lequel R est la chaîne latérale α de valine, isoleucine, leucine ou 5-méthylène-isoleucine ; et
Thz est un cycle thiazoline 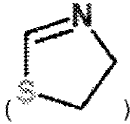 qui est couplé en 2' à V et couplé en 4' au carbone α de Leu^{[3]},
dans lequel aucun ou un ou plusieurs, des acides aminés Leu^{[3]}, Glu^{[4]}, Orn^{[7]}, Phe^{[9]}, His^{[10]} ou Asp^{[11} n'est/sont remplacé(s) par un autre résidu d'acide aminé qui peut être sélectionné parmi des acides aminés naturels ou non génétiquement codés, de préférence dans lequel ledit acide aminé naturel ou non génétiquement codé est sélectionné parmi leucine, thréonine, un acide, phénylalanine, arginine, histidine, lysine, asparagine, sérine, cystéine, homolysine, ornithine, l'acide diaminobutyrique (par exemple l'acide α,γ-diaminobutyrique), l'acide diaminopimélique, l'acide diaminopropionique, homoarginine, triméthylysine, triméthylornithine, l'acide 4-aminopipéridine-4-carboxylique, l'acide 4-amino-1- carbamimidoylpipéridine-4-carboxylique et 4-guanidinophénylalanine ;
(iii) sélectionné parmi la bacitracine A (A1 et/ou A2) et la bacitracine B (B1 et/ou B2) ; et/ou
(iv) complexé avec des cations métalliques divalents, par exemple Zn²⁺, Mg2+, Mn²⁺, et Co²⁺

3. Conjugué oligomère d'alginate-bacitracine selon la revendication 1 ou la revendication 2, dans lequel l'oligomère d'alginate présente un degré de polymérisation (DP), ou un degré de polymérisation moyen en nombre (DPn) de
(i) 4 à 50, 4 à 35, 4 à 30, 4 à 25, 4 à 22, 4 à 20, 4 à 18, 4 à 16 ou 4 à 14,
(ii) 6 à 50, 6 à 35, 6 à 30, 6 à 25, 6 à 22, 6 à 20, 6 à 18, 6 à 16 ou 6 à 14,
(iii) 8 à 50, 8 à 35, 8 à 30, 8 à 25, 10 à 25, 10 à 22, 10 à 20, 10 à 18, ou 10 à 15, ou
(iv) 20 à 50, 20 à 45, 20 à 40, 20 à 35, 20 à 30 ou 20 à 25, ou
(v) 30 à 50, 30 à 45, 30 à 40 ou 30 à 35.

4. Conjugué oligomère d'alginate-bacitracine selon l'une quelconque des revendications 1 à 3, dans lequel l'oligomère d'alginate présente au moins 70 % de résidus G, ou au moins 80 %, ou au moins 85 %, ou au moins 90 %, au moins 95 % de résidus G, de préférence dans lequel au moins 80 % des résidus G sont agencés sous forme de blocs G.

5. Conjugué oligomère d'alginate-bacitracine selon l'une quelconque des revendications 1 à 3, dans lequel l'oligomère d'alginate présente au moins 70 % de résidus M, ou au moins 80 %, ou au moins 85 %, ou au moins 90 %, au moins 95 % de résidus M, de préférence dans lequel au moins 80 % des résidus M sont agencés sous forme de blocs M.

6. Conjugué oligomère d'alginate-bacitracine selon l'une quelconque des revendications 1 à 5, dans lequel ladite liaison covalente directe fait partie d'un groupe ester, ester de carbonate, orthoester, cétal, hémi-acétal, éther, acétal, hémi-acétal, peroxy, méthylènedioxy, amide, amine, imine, imide, azide, azo, oxime, sulfure, disulfure, sulfinyle, sulfonyle, carbonothioyle, thioester, phosphine ou phosphodiester fonctionnel, de préférence partie d'un ester ou d'un amide.

7. Conjugué oligomère d'alginate-bacitracine selon l'une quelconque des revendications 1 à 5, dans lequel ledit lieur covalent est ou comprend des groupes moléculaires sélectionnés parmi :
(i) un acide aminé ou un peptide ;
(ii) un monosaccharide ou un oligosaccharide autre que guluronate ou mannuronate ou des polymères formés à partir de ceux-ci ;
(iii) un ribonucléotide ou un désoxyribonucléotide ;
(iv) un groupe alkyle, alcényle ou alcynyle à chaîne droite, ramifiée ou cyclique, substitué ou non substitué ;
(v) un groupe acétyle, succinyle, aconityle (cis ou trans), glutaryle, méthylsuccinyle, trimellityle cystéamine, pénicillamine, N-(2-mercaptopropionyl) glycine, d'acide 2-mercaptopropionique, homocystéine, d'acide 3-mercaptopropionique ou déamino-pénicillamine.

8. Conjugué oligomère d'alginate-bacitracine selon l'une quelconque des revendications 1 à 7, dans lequel ladite liaison covalente directe, un groupe fonctionnel contenant ladite liaison covalente ou ledit lieur moléculaire covalent est
(i) labile acide ;
(ii) sensible aux espèces réactives à l'oxygène ; et/ou
(iii) dégradé par une enzyme sécrétée par une bactérie ou une cellule immunitaire.

9. Conjugué oligomère d'alginate-bacitracine selon l'une quelconque des revendications 1 à 8, dans lequel ledit conjugué consiste en
(i) au moins un oligomère d'alginate lié de manière covalente à un antibiotique de classe bacitracine par l'intermédiaire d'une liaison ester formée à partir d'un groupe carboxyle sur l'alginate et le groupe hydroxyle sur la bacitracine, ou
(ii) au moins un oligomère d'alginate lié de manière covalente à un antibiotique de classe bacitracine par l'intermédiaire d'une liaison amide formée à partir d'un groupe carboxyle sur l'alginate et un groupe amine sur la bacitracine.

10. Composition pharmaceutique comprenant un conjugué oligomère d'alginate-bacitracine selon l'une quelconque des revendications 1 à 9 et un excipient, véhicule ou diluant pharmaceutiquement acceptable.

11. Procédé de préparation d'un oligomère d'alginate-bacitracine selon l'une quelconque des revendications 1 à 9, ledit procédé comprenant
(ia) la fourniture d'un oligomère d'alginate, dans lequel ledit oligomère d'alginate présente 2 à 50 résidus monomères, et un antibiotique de classe bacitracine et la formation d'une liaison covalente directe entre deux groupes moléculaires sur celui-ci, ou
(ib) la fourniture d'un oligomère d'alginate, d'un antibiotique de classe bacitracine et d'un lieur moléculaire covalent et la formation d'une liaison covalente directe entre deux groupes moléculaires sur l'oligomère d'alginate et la molécule lieuse et la formation d'une liaison covalente directe entre deux groupes moléculaires sur l'antibiotique de classe bacitracine et la molécule lieuse ; ou
(ic) la fourniture d'un oligomère d'alginate et d'un antibiotique de classe bacitracine dans lequel l'un ou les deux portent une molécule de type lieur moléculaire covalent liée de manière covalente à celui-ci et liant de manière covalente l'oligomère d'alginate à l'antibiotique de classe bacitracine par l'intermédiaire d'au moins l'une des molécules lieuses ; et éventuellement
(ii) la séparation d'au moins une portion du conjugué oligomère d'alginate-bacitracine du mélange réactionnel.

12. Procédé selon la revendication 11, ledit procédé comprenant
(i) la fourniture d'une solution aqueuse d'un oligomère d'alginate présentant un groupe carboxyle disponible dans lequel ledit oligomère d'alginate présente 2 à 50 résidus monomère
(ii) la mise en contact de ladite solution d'alginate avec du chlorhydrate de 1-éthyl-3-[3-diméthylaminopropyl]carbodiimide (EDC) en une quantité et dans des conditions suffisantes pour activer au moins un groupe carboxyle dans l'oligomère d'alginate ;
(iii) éventuellement la mise en contact dudit oligomère d'alginate à carboxyle activé avec un sulfo N-hydroxysuccinimide (sulfo-NHS) en une quantité et dans des conditions suffisantes pour former un ester sulfo-NHS aminoréactif ;
(iv) la mise en contact dudit oligomère d'alginate à carboxyle activé de l'étape (ii) ou l'ester sulfo-NHS aminoréactif de l'étape (iii) avec un antibiotique de classe bacitracine présentant un groupe amine primaire disponible en une quantité et dans des conditions suffisantes pour former une liaison amide entre l'oligomère d'alginate et l'antibiotique de classe bacitracine ; et
(v) la séparation d'au moins une portion du conjugué oligomère d'alginate-bacitracine du mélange réactionnel.

13. Procédé selon la revendication 11, ledit procédé comprenant
(i) la fourniture d'une solution d'un oligomère d'alginate présentant un groupe carboxyle disponible, dans lequel ledit oligomère d'alginate présente 2 à 50 résidus monomères, de préférence une solution (par exemple DMF et/ou DMSO) organique ;
(ii) la mise en contact de ladite solution d'alginate avec du dicyclohexylcarbodiimide (DCC) en une quantité et dans des conditions suffisantes pour former un intermédiaire O-acylisourée ;
(iii) la mise en contact dudit intermédiaire O-acylisourée avec un antibiotique de classe bacitracine présentant un groupe hydroxyle disponible et de la 4-N,N-diméthylaminopyridine (DMAP) en des quantités et dans des conditions suffisantes pour former une liaison ester entre l'oligomère d'alginate et l'antibiotique de classe bacitracine ; et
(iv) la séparation d'au moins une portion du conjugué oligomère d'alginate-bacitracine du mélange réactionnel ;
dans lequel les étapes (ii) et (iii) peuvent être réalisées simultanément.

14. Conjugué oligomère d'alginate-bacitracine selon l'une quelconque des revendication 1 à 9 ou composition pharmaceutique selon la revendication 10 pour utilisation dans le traitement de prévention d'une infection bactérienne, de préférence dans lequel l'infection bactérienne est
(i) dans une plaie, de préférence une plaie chronique ;
(ii) une infection respiratoire chez un sujet atteint d'un trouble ou d'une maladie respiratoire sous-jacent, de préférence sélectionné parmi la mucoviscidose, une BPCO/MPOC, ou l'asthme;
(iii) une infection liée à un dispositif associée à des dispositifs médicaux implantables ou prothétiques ; ou
(iv) une infection systémique ou une infection à loci multiples dans ou sur le sujet.

15. Conjugué oligomère d'alginate-bacitracine ou composition pharmaceutique pour utilisation selon la revendication 14, dans lequel l'infection est une infection bactérienne à gram négatif.
